(19) Europäisches Patentamt / European Patent Office / Office européen des brevets

(11) **EP 3 899 038 B1**

(12) **EUROPEAN PATENT SPECIFICATION**

(45) Date of publication and mention
of the grant of the patent:
**21.05.2025 Bulletin 2025/21**

(51) International Patent Classification (IPC):
**C12Q 1/6844** *(2018.01)* **B01L 3/00** *(2006.01)*
**C12Q 1/6816** *(2018.01)*

(21) Application number: **19899251.3**

(52) Cooperative Patent Classification (CPC):
(C-Sets available)
**C12Q 1/6816** (Cont.)

(22) Date of filing: **20.12.2019**

(86) International application number:
**PCT/US2019/067967**

(87) International publication number:
**WO 2020/132515 (25.06.2020 Gazette 2020/26)**

(54) **RATIOMETRIC FLUORESCENCE CODING METHOD FOR MULTIPLEX NUCLEIC ACID AMPLIFICATION ASSAYS**

RATIOMETRISCHES FLUORESZENZKODIERUNGSVERFAHREN FÜR MULTIPLEXNUKLEINSÄUREAMPLIFIKATIONSTESTS

PROCÉDÉ DE CODAGE PAR FLUORESCENCE RATIOMÉTRIQUE POUR DOSAGES D'AMPLIFICATION D'ACIDE NUCLÉIQUE MULTIPLEX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2018 US 201862784100 P**

(43) Date of publication of application:
**27.10.2021 Bulletin 2021/43**

(73) Proprietor: **The Johns Hopkins University Baltimore, Maryland 21218 (US)**

(72) Inventors:
 • **WANG, Tza-Huei Jeff**
 **Timonium, Maryland 21093 (US)**
 • **ZHANG, Ye**
 **Baltimore, Maryland 21218 (US)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(56) References cited:
WO-A1-2015/071445 WO-A1-2016/057552
WO-A2-2010/101926 WO-A2-2010/129787
CN-B- 103 952 146 US-A1- 2011 287 976
US-A1- 2012 135 874

• BEH CYRUS W. ET AL: "Direct Interrogation of DNA Content Distribution in Nanoparticles by a Novel Microfluidics-Based Single-Particle Analysis", NANO LETTERS, vol. 14, no. 8, 31 July 2014 (2014-07-31), US, pages 4729 - 4735, XP055952953, ISSN: 1530-6984, DOI: 10.1021/ nl5018404
• YE ZHANG, LIBEN CHEN, KUANGWEN HSIEH, TZA-HUEI WANG: "Ratiometric Fluorescence Coding for Multiplex Nucleic Acid Amplification Testing", ANALYTICAL CHEMISTRY, AMERICAN CHEMICAL SOCIETY, US, vol. 90, no. 20, 16 October 2018 (2018-10-16), US, pages 12180 - 12186, XP055714044, ISSN: 0003-2700, DOI: 10.1021/acs.analchem.8b03266

(52) Cooperative Patent Classification (CPC): (Cont.)

C-Sets
**C12Q 1/6816, C12Q 2521/501, C12Q 2525/107,
C12Q 2525/113, C12Q 2525/155, C12Q 2525/161,
C12Q 2525/307, C12Q 2531/113, C12Q 2531/125,
C12Q 2533/107, C12Q 2537/143, C12Q 2563/107,
C12Q 2565/101, C12Q 2565/102, C12Q 2565/629**

## Description

### CROSS-REFERENCE OF RELATED APPLICATION

This application claims priority to U.S. Provisional Application No. 62/784,100 filed December 21, 201 8.

### BACKGROUND

1. Technical Field

Aspects of the invention relate to a method for multiplexed detection of a nucleic acid sequence in a sample comprising the use of a plurality of oligonucleotide target-specific probes (TSPs) configured to bind to a distinct target nucleic acid sequence.

2. Discussion of Related Art

Nucleic acid amplification testing (NAAT) has emerged as a popular technique for the diagnosis of many diseases including cancer [1, 2], genetic disorders[3], mitochondrial disorders [4], and infectious diseases[5]. Indeed, NAAT methods such as real-time polymerase chain reaction (PCR) [6, 7], ligase chain reaction (LCR) [8, 9], and nucleic acid sequence-based amplification (NASBA) [10, 11] have been widely employed for nucleic acid detection because of their high sensitivity, specificity, and rapid turn-around-time. In addition, a number of different approaches can be coupled with NAAT to achieve multiplexed detection of multiple targets in a single assay, which is a particularly useful capability for clinical diagnosis of diseases [5, 12]. Among these approaches, which range from differentiating the melting temperatures of NAAT amplicons [13, 14] to assigning targets with probes that have distinct lengths (i.e., multiplexed ligation-dependent amplification [15, 16]), the most commonly employed strategy has been using a target-specific, fluorescently-labeled oligonucleotide probe for each target nucleic acid sequence. While this "one-color-one-target" approach is straightforward and effectively leverages the mature fluorescence detection infrastructure associated with NAAT methods, it unfortunately has a limited capacity of multiplexing because spectral overlaps between fluorophores restrict the number of fluorophores that can be used within a single assay. As such, there remains a need for fluorescence-based approaches that extend beyond the one-color-one-target restriction and expand the multiplexing capacity of NAAT.

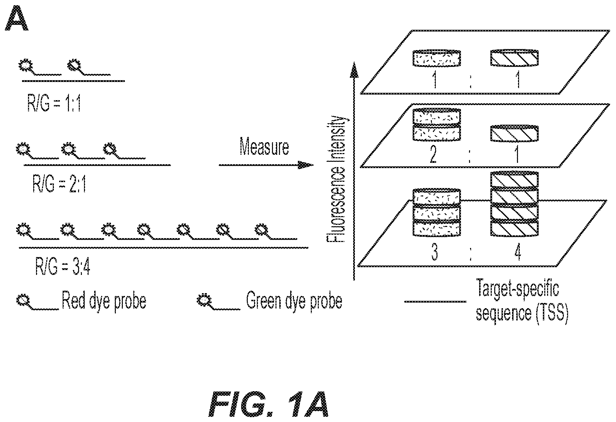

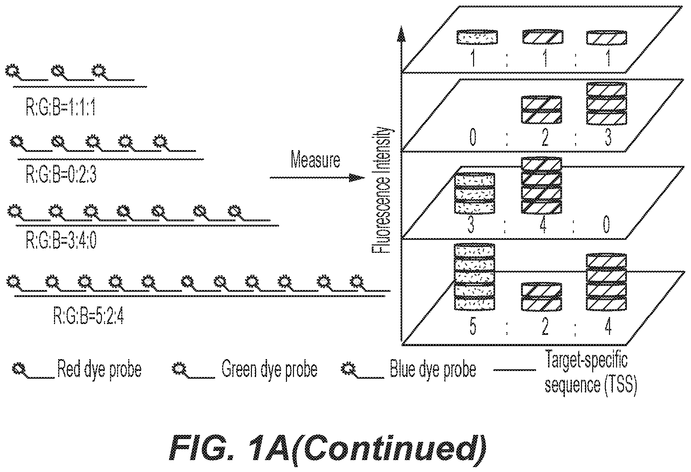

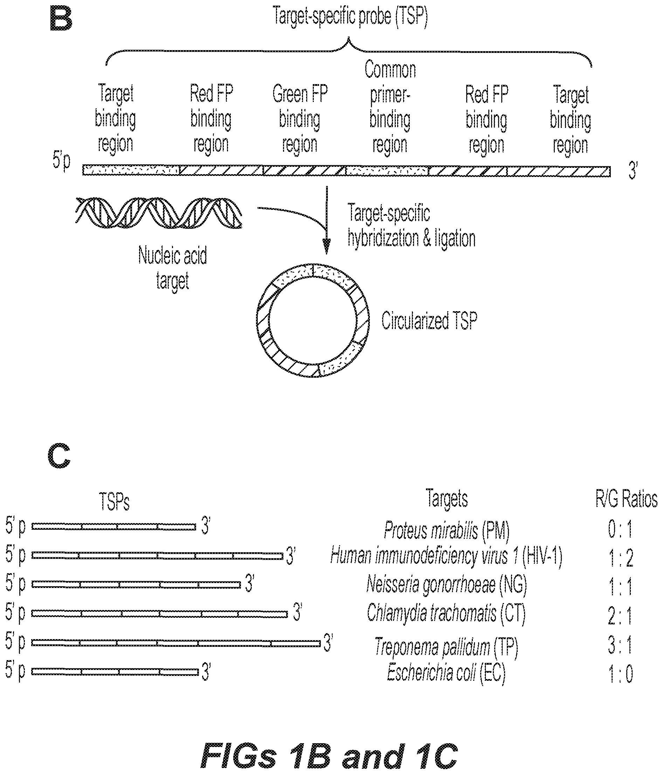

A natural and promising extension to the one-color-one-target approach is using a unique combination of multiple fluorophores to encode each target sequence. A handful of strategies for demonstrating this approach have been reported. For example, 15-plex detection using only 4 fluorophores has been achieved through a strategy called Multicolor Combinational Probe Coding (MCPC) [17, 18]. To achieve 15-plex detection via MCPC, however, 32 different fluorescently-labeled probes were synthesized, which escalated the cost of this strategy. Moreover, the probe design principle in MCPC capped its multiplexing capacity to 15 when using 4 fluorophores; and as spectral overlaps between fluorophores make it difficult to incorporate additional fluorophores, MCPC had likely reached its maximum multiplexing capacity. Another strategy, which was coined binary-scheme mathematical theory, employed each fluorescence color as a "digit" in the binary code and assigned each target sequence with a unique binary code of at least two colors/digits[19]. Unfortunately, even when using 4 fluorophores, this strategy still only achieved 4-plex detection. Therefore, despite the potential, effective strategies for leveraging combinations of multiple fluorophores to achieve multiplexed NAAT remain underdeveloped to date. The closest prior art is probably the publication by Ye Zhang et al., "Ratiometric Fluorescence Coding for Multiplex Nucleic Acid Chemistry", volume 90, number 8, of October 16th 2018, pages 12180 - 12186, detailing a multiplexed detection approach termed ratiometric fluorescence coding" where specific ratios between two standard fluorophores are used. Padlock probes are used and labelled with two different fluorophores. The principle of this technique is visualized with Figure 1A. In the presence of the corresponding target, the padlock probe is ligated to a closed circle for subsequent amplification. The nucleic acid amplification assay that follows, is described to be RCA whereby long ssDNAs are generated, comprising thousands of tandem repeats complementary to the padlock probe sequences, serving as binding sites for fluorophores-labeled PNA probes. The linear padlock probes apart from the "target binding regions" comprise furthermore specific binding sites for the differently labelled probes ("Red probe site" and "Green probe site") as well as "general primer sites", the latter equivalent to the "common primer binding region" as claimed in the first claim, all of which being visualized in Figure 1B. This document however does not introduce molecular beacon - fluorescent probes.

### BRIEF DESCRIPTION OF THE DRAWINGS

**FIGs 1A-1D** are schematics providing an overview of Ratiometric Fluorescence Coding for multiplexed nucleic acid detection, according to an embodiment of the invention;

**FIGs 2A and 2B** show results of six independent experiments for detecting the six infectious disease-related synthetic targets, according to an embodiment of the invention;

**FIGs 3A and 3B** show results of an assay detecting four infectious disease-related synthetic targets, according to an embodiment of the invention;

**FIGs 4A - 4C** show results of various assays testing the sensitivity of a ratiometric fluorescence coding method according to an embodiment of the invention;

**FIG. 5** is a schematic showing a digital microfluidic platform for Ratiometric Fluorescence coding assay using PCR, according to an embodiment of the invention;

**FIG. 6** is a schematic showing a digital microfluidic platform for Ratiometric Fluorescence coding assay using HRCA, according to an embodiment of the invention;

**FIG. 7** is a schematic showing a digital droplet platform for Ratiometric Fluorescence coding assay using PCR, according to an embodiment of the invention;

**FIG. 8** is a schematic showing a digital droplet platform for Ratiometric Fluorescence coding assay using HRCA, according to an embodiment of the invention;

**FIGs 9A and 9B** are data graphs showing that the digital droplet platform for Ratiometric Fluorescence coding assay using HRCA has been verified using six TSPs and STD synthetic targets, according to an embodiment of the invention;

**FIG. 10** is a schematic showing single-molecule Ratiometric Fluorescence Coding assay, according to an embodiment of the invention;

**FIG. 11** is a series of data graphs showing that the single-molecule Ratiometric Fluorescence Coding assay was verified using four TSPs and STD synthetic targets, according to an embodiment of the invention;

**FIG. 12** is a gel showing amplified nucleic acids, according to an embodiment of the invention;

**FIG. 13** is a schematic showing a pair of probes binding to a target nucleic acid sequence, according to an embodiment of the invention;

**FIG. 14** is a schematic illustration of ligation-based multiplex nucleic acid detection with fluorescence coding according to an embodiment of the invention;

**FIG. 15** is a schematic showing experimental steps for demonstrating the performance of a method according to an embodiment of the invention;

**FIGs 16A and 16B** show results for a 10-plex detection method according to an embodiment of the invention;

**FIG. 17** is a series of data plots showing seven distinct fluorescent patterns generated from ligation products according to an embodiment of the invention; and

**FIG. 18** is a series of data plots showing simultaneous detection of seven different targets with ligation-based digital PCR analysis according to an embodiment of the invention.

## DETAILED DESCRIPTION

An embodiment of the invention relates to a method for multiplexed detection of a nucleic acid sequence in a sample including the steps of: obtaining a plurality of oligonucleotide target-specific probes (TSPs), where each of the TSPs is configured to bind to a distinct target nucleic acid sequence, and where each of the TSPs includes: at least one target-binding region configured to bind to at least a portion of the distinct target nucleic acid sequence; at least one common primer-binding region; and one or more copies of a first fluorescent probe (FP) binding region and one or more copies of a second FP binding region, where a predetermined ratio of the one or more copies of the first FP binding region to the one or more copies of the second FP binding region is indicative of the distinct target nucleic acid sequence the TSP is configured to bind to; contacting the plurality of TSPs with the nucleic acid sequence in the sample such that at least one of the TSPs binds to at least a portion of the nucleic acid sequence and such that a TSP-nucleic acid sequence complex is formed; ligating the at least one of the TSPs bound to at least a portion of the nucleic acid sequence such that a ligated TSP is formed; generating target-specific oligonucleotide sequences (TSSs) by a nucleic acid amplification assay, and where the ligated TSP is a template for the nucleic acid amplification assay; contacting the TSSs with a plurality of differently labeled fluorescent probes (FPs) such that a TSS-FP complex comprising the TSS and at least one of the first FP and the second FP is generated; measuring a fluorescence ratio; and identifying the nucleic acid sequence based on the florescence ratio.

An embodiment of the invention relates to the method above, where each of the TSPs further includes: a first target-binding region at a first end of the TSP, wherein the first target-binding region binds to at least a portion of the distinct target nucleic acid sequence; and a second target-binding region at a second end of the TSP, wherein the second target-binding region binds to at least a portion of the distinct target nucleic acid sequence. In such an embodiment, the ligating the at least one of the TSPs bound to at least a portion of the nucleic acid sequence results in a circularized TSP, and the

EP 3 899 038 B1

circularized TSP is the template for the nucleic acid amplification assay.

An embodiment of the invention relates to the method above, where the plurality of oligonucleotide target-specific probes (TSPs), includes a plurality of TSP pairs configured to bind to a distinct target nucleic acid sequence, and wherein each pair of the plurality of TSP pairs includes: a first TSP comprising: a target-specific binding region configured to bind to at least a first portion of the distinct target nucleic acid sequence, wherein the first target-specific binding region is located at a 3' end of the first TSP; at least one common primer-binding region; and one or more copies of a first fluorescent probe (FP) binding region; and a second TSP including: a target-specific binding region configured to bind to at least a second portion of the distinct target nucleic acid sequence, wherein the target-specific binding region is located at a 5' end of the second TSP; at least one common primer-binding region; and one or more copies of a second fluorescent probe (FP) binding region. In such an embodiment, the first portion of the distinct target nucleic acid sequence and the second portion of the distinct target nucleic acid sequence are adjacent, and a predetermined ratio of the one or more copies of the first FP binding region to the one or more copies of the second FP binding region is indicative of the distinct target nucleic acid sequence the pair is configured to bind to.

An embodiment of the invention relates to the method above, where the FPs comprise linear oligonucleotide probes labeled with fluorophores and molecule beacons (MBs) that are hairpin shaped oligonucleotide probes labeled with fluorophores and quenchers, and where MBs fluorescence is quenched in a native state and restored upon hybridization to the TSSs.

An embodiment of the invention relates to the method above, where the FPs include oligonucleotides sequences comprising one or more of nucleic acids, nucleic acid analogues including peptide nucleic acids (PNAs), and locked nucleic acids (LNAs).

An embodiment of the invention relates to the method above, where the nucleic acid amplification assay is selected from the list consisting of rolling circle amplification (RCA), hyperbranched rolling circle amplification (HRCA), and polymerase chain reaction (PCR).

An embodiment of the invention relates to the method above, where the sample includes one unidentified nucleic acid target sequence out of multiple candidates.

An embodiment of the invention relates to the method above, further including removing TSPs that are not bound to at least a portion of the nucleic acid sequence following the ligating of the at least one of the TSPs bound to at least a portion of the nucleic acid sequence.

An embodiment of the invention relates to the method above, further including removing FPs not hybridized to the TSSs comprising the use a purification spin column.

An embodiment of the invention relates to the method above, further including the use of a single molecule detection (SMD) system, and measuring a photon count of each fluorescence color of a single TSS-FP complex including the use of cylindrical illustration confocal spectroscopy (CICS).

An embodiment of the invention relates to the method above, where the SMD system comprises a microfluidic chip having: a gas permeable silicone material comprising polydimethylsiloxane (PDMS); a transport chamber comprising at least 2 parallel flow channels, each flow channel having a dimension of about 5 $\mu$m $\times$ 0.5 $\mu$m (width $\times$ height); and a filter array at an inlet to reduce flow channel clogging. In such an embodiment, the microfluidic chip is used for measuring fluorescence of the TSS-TPs complex.

An embodiment of the invention relates to the method above, where the sample is driven through the microfluidic chip using a nitrogen pressure source, and the nitrogen pressure source is regulated by a series of precision gas regulators.

An embodiment of the invention relates to the method above, where the photon count of each fluorescence color of the TSS- FP complex is measured on CICS, and the nucleic acid sequence is identified by a ratio of measured fluorescence photon counts.

An embodiment of the invention relates to the method above, further including: loading a plurality of ligated TSPs, a nucleic acid amplification reaction mixture, and FPs onto an array of discrete reaction receptacles, such that each reaction receptacle contains up to one ligated TSP; generating TSSs in each reaction receptacle by nucleic acid amplification using the ligated TSP as a template; binding the TSSs with a plurality of differently labeled FPs in each reaction receptacle; measuring a fluorescence ratio in each of the reaction receptacles; and identifying the nucleic acid sequence based on the florescence ratio.

An embodiment of the invention relates to the method above, where the sample contains a plurality of nucleic acid sequences.

An embodiment of the invention relates to the method above, where the array of discrete reaction receptacles are located on a microfluidic chip, or the array of discrete reaction receptacles are a plurality of droplets.

An embodiment of the invention relates to the method above, where the chip further includes: a microfluidic flow chamber comprising one or more flow channels; and a plurality of picowells with dimensions in the range of 100pL to 10nL. In such an embodiment, the one or more flow channels are in contact with the plurality of picowells.

An embodiment of the invention relates to the method above, further including loading the plurality of ligated

5

TSPs, nucleic acid amplification reaction mixture and a plurality of differently labeled MBs onto the one or more flow channels of the microfluidic chip such that each of the plurality of picowells contains up to one ligated TSP; injecting fluid comprising oil and polydimethylsiloxane (PDMS) into the one or more flow channels but not into the plurality of picowells, such that a digital reaction well is formed; generating TSSs in each of the plurality of picowells containing up to one ligated TSP by nucleic acid amplification using the ligated TSP as a template; binding the TSSs with the differently labeled MBs; measuring a fluorescence ratio; and identifying the nucleic acid sequence based on the florescence ratio.

An embodiment of the invention relates to the method above, where each of the plurality of droplets have a volume of between about 5pL to 1nL.

An embodiment of the invention relates to the method above, where the plurality of droplets are generated on a microfluidic chip including: a droplet generation module comprising flow channels, microvalves and a flow focusing junction; and a droplet measurement module comprising flow channels, microvalves and a flow constriction channel.

An embodiment of the invention relates to the method above, where a custom confocal microscope system is used for measuring a fluorescence intensity in the plurality of droplets, the custom confocal microscope including: a 488nm laser and a 545 nm laser; a plurality of dichroic mirrors to combine two laser beams; a 40× microscope objective to focus a laser beam and to collect an emitted fluorescence signal from the plurality of droplets; a plurality of dichroic mirrors and band-pass filters to spectrally separate a desired emission fluorescence signal; and at least 2 two avalanche photodiodes (APDs) to collect fluorescence data.

An embodiment of the invention relates to the method above, further including: loading a mixture comprising the plurality of ligated TSPs, nucleic acid amplification reaction mixture, and FPs into a droplet generation module on the chip; loading fluid comprising an oil and a surfactant into the droplet generation module simultaneously such that the mixture is sheared into a plurality of droplets, and wherein each droplet contains up to one ligated TSP; collecting the plurality of droplets; incubating the collected plurality of droplets on a thermal cycler and generating TSSs by nucleic acid amplification using the ligated TSP as a template; binding the TSSs with the differently labeled FPs; loading the plurality of droplets into a droplet measurement module on the chip; measuring a fluorescence ratio using a custom confocal microscope system; and identifying the nucleic acid sequence based on the measured fluorescence ratio.

An embodiment of the invention relates to the method above, where each of the TSPs further includes one or more copies of a third fluorescent probe (FP) binding region, wherein a predetermined ratio of the one or more copies of the first FP binding region to the one or more copies of the second FP binding region and to the one or more copies of the third FP binding region is indicative of the distinct target nucleic acid sequence the TSP is configured to bind to.

An embodiment of the invention relates to a kit for multiplexed detection of a nucleic acid sequence in a sample including: a plurality of oligonucleotide target-specific probes (TSPs), wherein each of the TSPs is configured to bind to a distinct target nucleic acid sequence, and wherein each of the TSPs includes: at least one target-binding region configured to bind to at least a portion of the distinct target nucleic acid sequence; at least one common primer-binding region; and one or more copies of a first fluorescent probe (FP) binding region and one or more copies of a second FP binding region, where a predetermined ratio of the one or more copies of the first FP binding region to the one or more copies of the second FP binding region is indicative of the distinct target nucleic acid sequence the TSP is configured to bind to; and instructions for determining the identity of the nucleic acid sequence based on a measured fluorescence ratio.

An embodiment of the invention relates to the kit above, where each of the TSPs further includes: a first target-binding region at a first end of the TSP, wherein the first target-binding region binds to at least a portion of the distinct target nucleic acid sequence; and a second target-binding region at a second end of the TSP, wherein the second target-binding region binds to at least a portion of the distinct target nucleic acid sequence.

An embodiment of the invention relates to the kit above, where the plurality of oligonucleotide target-specific probes (TSPs), comprises a plurality of TSP pairs configured to bind to a distinct target nucleic acid sequence, and where each pair of the plurality of TSP pairs includes: a first TSP having: a target-specific binding region configured to bind to at least a first portion of the distinct target nucleic acid sequence, where the first target-specific binding region is located at a 3' end of the first TSP; at least one common primer-binding region; and one or more copies of a first fluorescent probe (FP) binding region; and a second TSP having: a target-specific binding region configured to bind to at least a second portion of the distinct target nucleic acid sequence, wherein the target-specific binding region is located at a 5' end of the second TSP; at least one common primer-binding region; and one or more copies of a second fluorescent probe (FP) binding region. In such an embodiment, the first portion of the distinct target nucleic acid sequence and the second portion of the distinct target nucleic acid sequence are adjacent, and a predetermined ratio of the one or more copies of the first FP binding region to the one or more copies of the second FP binding region is indicative of the distinct target nucleic acid sequence the pair is configured to bind to.

An embodiment of the invention relates to the kit above, further including a primer which binds to the common primer-binding region.

An embodiment of the invention relates to the kit above, further including reagents for conducting a nucleic acid amplification assay.

An embodiment of the invention relates to the kit above, where the reagents for conducting a nucleic acid amplification assay are reagents suitable for conducting a rolling circle amplification assay, a hyperbranched rolling circle amplification assay, or a polymerase chain reaction assay.

An embodiment of the invention relates to the kit above, further including the first FP and the second FP.

An embodiment of the invention relates to the kit above, where the first FP and the second FP include linear oligonucleotide probes labeled with fluorophores and molecule beacons (MBs) that are hairpin shaped oligonucleotide probes labeled with fluorophores and quenchers, and wherein MBs fluorescence is quenched in a native state and restored upon hybridization to the TSSs.

An embodiment of the invention relates to the kit above, where the first FP and the second FP comprise oligonucleotides sequences comprising one or more of nucleic acids, nucleic acid analogues including peptide nucleic acids (PNAs), and locked nucleic acids (LNAs).

An embodiment of the invention relates to the kit above, further including an enzyme for removing or inactivating oligonucleotide target-specific probes that do not bind to the nucleic acid.

An embodiment of the invention relates to a method for multiplexed detection of nucleic acid targets termed Ratiometric Fluorescence Coding comprising the steps of: creating a plurality of target-specific oligonucleotide probes (TSPs), wherein each TSP consists of (1) two target-binding regions, each at either end of the TSP, (2) one or two common primer-binding regions, (3) one or more fluorescent probe (FP) binding regions that can be repeated for binding multiple copies of one or more differently labeled FPs; ligating the TSPs in a sample containing nucleic acid targets to generate circularized TSPs; generating target-specific oligonucleotide sequences (TSSs) by nucleic acid amplification using the circularized TSPs as templates and one or two common primers; binding the TSSs with a plurality of differently labeled FPs; identifying each nucleic acid target by a predetermined ratio of fluorescence intensity generated from the one or more FPs.

An embodiment relates to the method above, further including a step of mixing a biological sample containing unidentified nucleic acid targets with all TSPs for probe-target hybridization and ligation.

An embodiment relates to the method above, where FPs of the same fluorescence labeling have the same oligonucleotide sequences.

An embodiment relates to the method above, where FPs include linear oligonucleotide probes labeled with fluorophores and molecule beacons (MBs) that are hairpin shaped oligonucleotide probes labeled with fluorophores and quenchers wherein fluorescence on MBs is quenched in native state and restored upon hybridization to the TSSs.

An embodiment relates to the method above, where the oligonucleotide sequences of FPs comprise of nucleic acids, nucleic acid analogues including peptide nucleic acids (PNAs) and locked nucleic acids (LNAs), or a combination of thereof.

An embodiment relates to the method above, where the nucleic acid amplification for generating TSSs includes one or more of rolling circle amplification (RCA), hyperbranched rolling circle amplification (HRCA), polymerase chain reaction (PCR), etc.

An embodiment relates to a method for identifying one nucleic acid target out of multiple possible candidates using the Ratiometric Fluorescence Coding above comprising the steps of: creating a plurality of TSPs as above; ligating the TSPs in a sample containing nucleic acid targets to generate circularized TSPs; generating TSSs by nucleic acid amplification using the circularized TSPs as templates and one or two common primers; binding the TSSs with a plurality of differently labeled FPs; identifying each nucleic acid target by a predetermined ratio of fluorescence intensity generated from the one or more FPs.

An embodiment relates to the method above, where the biological sample contains one unidentified nucleic acid target out of multiple candidates.

An embodiment relates to the method above, where FPs of the same fluorescence labeling have the same oligonucleotide sequences.

An embodiment relates to the method above, where FPs include linear oligonucleotide probes labeled with fluorophores and MBs.

An embodiment relates to the method above, where the oligonucleotide sequences of FPs comprise nucleic acids, nucleic acid analogues including PNAs and LNAs, or a combination of thereof.

An embodiment relates to the method above, where the nucleic acid amplification for generating TSSs include RCA, HRCA, PCR, etc.

An embodiment relates to the method above, further comprising a step of removing extra linear TSPs that are not circularized after ligation reaction using exonucleases if HRCA is used for generating TSSs.

An embodiment relates to the method above, further comprising a step of removing non-hybridized, excess FPs using purification spin columns if linear oligonucleotide probes are used as FPs.

An embodiment relates to the method above, where the intensity of each fluorescence color of the TSS- FPs complexes are measured, and each nucleic acid target is identified by the ratio of measured fluorescence intensities.

An embodiment relates to a method for implementing the Ratiometric Fluorescence Coding above onto a single molecule detection (SMD) system for multiplexed nucleic acid detection comprising the steps of: creating a plurality of TSPs above; ligating the TSPs in a sample containing nucleic acid targets to generate circularized TSPs; generating TSSs by nucleic acid amplification using the circularized TSPs as templates and one or two common primers; binding the TSSs with a plurality of differently labeled FPs; measuring photon counts of each fluorescence color of single TSS-FPs complex using cylindrical illustration confocal spectroscopy (CICS); identifying each nucleic acid target by a predetermined ratio of fluorescence intensity generated from the one or more FPs.

An embodiment relates to the method above, where the biological sample contains one or more unidentified nucleic acid targets.

An embodiment relates to the method above, where FPs of the same fluorescence labeling have the same oligonucleotide sequences.

An embodiment relates to the method above, where FPs include linear oligonucleotide probes labeled with fluorophores and MBs.

An embodiment relates to the method above, where the oligonucleotide sequences of FPs comprise of nucleic acids, nucleic acid analogues including PNAs and LNAs, or a combination of thereof.

An embodiment relates to the method above, where the nucleic acid amplification for generating TSSs include RCA, HRCA, PCR, etc.

An embodiment relates to the method above, further comprising a step of removing extra linear TSPs that are not circularized after ligation reaction using exonucleases if HRCA is used for generating TSSs.

An embodiment relates to the method above, further comprising a step of removing non-hybridized, excess FPs using purification spin columns if linear oligonucleotide probes are used as FPs.

An embodiment relates to the method above, where a microfluidic chip made of gas permeable silicone material comprising polydimethylsiloxane (PDMS) is used for fluorescence measurement of TSS-TPs complex on CICS comprising: a transport chamber comprising ten parallel flow channels wherein each channel is $5\,\mu m \times 0.5\,\mu m$ (w $\times$ h); a filter array at the inlet to reduce channel clogging.

An embodiment relates to the method above, where a custom-built SMD instrument termed cylindrical illumination confocal spectroscopy (CICS) is used for fluorescence measurement, where an 488nm Ar-ion laser and a 633nm He-Ne laser are combined using a dichroic mirror and coupled by passing through an optical fiber; where the output from the fiber are expanded using additional shaping optics and focused into a light sheet using a cylindrical lens; where the laser excitation sheet is focused into the center the flow channel on the microfluidic chip of claim 3.2 using a 100$\times$ oil immersion microscope objective for fluorescence excitation; where emitted fluorescence signals from the template-probe complex is collected by the same objective and a dichroic mirror is used to separate the excitation light and emission fluorescence; where a confocal aperture (600 $\times$ 150 $\mu m$) is used to spatially filter out out-of-plane light; where desired fluorescence signals are spectrally separated by dichroic mirrors and band-pass filters and detected on two avalanche photodiodes (APDs); where fluorescence data from APDs is collected and processed using custom software written in Labview program.

An embodiment relates to the method above, an embodiment relates to the method above, where samples are driven through the microfluidic chip above using a nitrogen pressure source controlled by a series of precision gas regulators.

An embodiment relates to the method above, where Piezoelectric stages are used to focus the CICS laser excitation sheet into the center of the microfluidic chip.

An embodiment relates to the method above, where each single molecule trace data is collected with a 0.1 ms bin time.

An embodiment relates to the method above, where the photon counts of each fluorescence color of the TSS-FPs complexes are measured on CICS, and nucleic acid targets are identified by the ratios of measured fluorescence photon counts.

An embodiment relates to the method above, further including use of an array of discrete reaction receptacles for multiplexed detection of nucleic acid targets and further including the steps of: creating a plurality of TSPs above; ligating the TSPs in a sample containing nucleic acid targets to generate circularized TSPs; loading circularized TSPs with nucleic acid amplification reaction mixture and FPs onto an array of discrete reaction receptacles so that each reaction receptacle contains no more than one copy of the circularized TSP; generating TSSs in each reaction receptacle by nucleic acid amplification using the circularized TSPs as templates and one or two common primers; binding the TSSs with a plurality of differently labeled FPs in each reaction receptacle; measuring intensity of each fluorescence color in each reaction receptacle; and identifying each nucleic acid target by a predetermined ratio of fluorescence intensity generated from the one or more FPs.

An embodiment relates to the method above, where the biological sample contains one or more unidentified nucleic acid targets.

An embodiment relates to the method above, where FPs of the same fluorescence labeling have the same oligonucleotide sequences.

An embodiment relates to the method above, where the oligonucleotide sequences of FPs comprise of nucleic acids, nucleic acid analogues including PNAs and LNAs, or a combination of thereof.

An embodiment relates to the method above, where the nucleic acid amplification for generating TSSs include RCA, HRCA, PCR, etc.

An embodiment relates to the method above, further comprising a step of removing extra linear TSPs that are not circularized after ligation reaction using exonucleases if HRCA is used for generating TSSs.

An embodiment relates to the method above, where the array of discrete reaction receptacles include a chip, droplets, etc.

An embodiment relates to the method above, where the chip includes a microfluidic flow chamber comprising one or more flow channels; and a plurality of picowells with dimensions in the range of 100pL to 10nL wherein the flow channels are in contact with the picowells.

An embodiment relates to the method above, where the chip is made of gas permeable silicone material comprising polydimethylsiloxane (PDMS).

An embodiment relates to the method above, where the chip further includes an external pump to drive continuous, unidirectional, or bidirectional fluid through the flow channels; and a digital analysis system comprises a thermal cycler for performing nucleic acid amplification, an optical setup for fluorescence measurement and a custom program for analyzing results written in Matlab.

An embodiment relates to the method above, where the chip system above is coupled with the Ratiometric Fluorescence Coding above for multiplexed detection comprising the steps of: loading the mixture of circularized TSPs, nucleic acid amplification reaction mixture and a plurality of differently labeled MBs onto the chip through the one or more flow channels wherein each picowell contains either 0 to 1 circularized TSPs; injecting fluid comprising oil and PDMS into the one or more flow channels but not the picowells to enclose the picowells forming digital reaction wells; generating TSSs in each picowell by nucleic acid amplification using the circularized TSPs as templates and one or two common primers; binding the TSSs with the differently labeled MBs in each picowell; measuring intensity of each fluorescence color in each picowell; identifying each nucleic acid target by the ratios of measured fluorescence intensities.

An embodiment relates to the method above, comprising a liquid mixture in oil wherein the mixture comprises circularized TSPs (before amplification) or TSSs (after amplification), nucleic acid amplification reaction mixture and a plurality of differently labeled MBs.

An embodiment relates to the method above, where the droplets have dimensions in the range of 5pL to 1nL.

An embodiment relates to the method above, where the droplets are generated by a PDMS chip comprising: a droplet generation module comprising flow channels, microvalves and a flow focusing junction; and a droplet measurement module comprising flow channels, microvalves and a flow constriction channel.

An embodiment relates to the method above, where a custom-built confocal microscope system is used for droplet fluorescence measurement comprising a 488nm laser and a 545 nm laser; dichroic mirrors to combine two laser beams; a 40× microscope objective to focus the laser beam into the center of the constriction channel on the chip above and collect emitted fluorescence signals from the droplets; dichroic mirrors and band-pass filters to spectrally separate desired emission fluorescence signals; and two APDs to collect fluorescence data.

An embodiment relates to the method above, where the droplets above are used for multiplexed detection further comprising a chip of claim above; an external pump to drive continuous, unidirectional, or bidirectional fluid through the flow channels; a thermal cycler for performing nucleic acid amplification; a custom-built confocal microscope system as described above; a custom software for data collection written in Labview; and a custom program for data analyzing written in Matlab.

An embodiment relates to the method above and the droplet system above coupled with the Ratiometric Fluorescence above for multiplexed detection comprising the steps of: injecting the mixture of circularized TSPs, nucleic acid amplification reaction mixture and a plurality of differently labeled MBs into the droplet generation module on the chip; injecting fluid comprising oil and surfactant into the droplet generation module at the same time to shear the mixture into droplets wherein each droplet contains either 0 to 1 circularized TSP; collecting droplets in a tube; incubating the tube containing droplets on thermal cycler and generating TSSs in each droplet by nucleic acid amplification using the circularized TSPs as templates and one or two common primers; binding the TSSs with the differently labeled MBs in each droplet; reinjecting the droplets into the droplet measurement module on the chip for fluorescence measurement; measuring intensity of each fluorescence color in each droplet using the custom-built confocal microscope system above; and identifying nucleic acid targets the ratios of measured fluorescence intensities.

An embodiment of the invention relates to a library of target-specific oligonucleotide probes comprising a plurality of oligonucleotide target-specific probes, wherein each of the plurality of oligonucleotide target-specific probes comprises: a first target-binding region for binding to at least a portion of a target nucleic acid sequence; a second binding region for binding to at least a portion of the target nucleic acid sequence; a general primer nucleotide sequence; and one or more copies of a first nucleic acid sequence for binding a first fluorescent molecular probe and one or more copies of a second nucleic acid sequence for binding a second fluorescent molecular probe. In such an embodiment, the first binding region is at a 5' end of the oligonucleotide target-specific probe and the second target-binding region is at a 3' end of the oligonucleotide target-specific probe, the general primer nucleotide sequence is conserved by all of the oligonucleotide target-specific probes, and a predetermined ratio of the one or more copies of the first nucleic acid sequence to the one or more copies of the second nucleic acid sequence is indicative of the distinct nucleic acid sequence the oligonucleotide target-specific probe is configured to bind to.

An embodiment of the invention relates to a method for determining the identity of a nucleic acid sequence in a sample comprising: obtaining a probe solution comprising a plurality of oligonucleotide target-specific probes from the library of above; creating a first reaction mixture by contacting the sample with the probe solution, wherein the nucleic acid binds to a oligonucleotide target-specific probe from the library and wherein the binding of the nucleic acid sequence to the oligonucleotide target-specific probe results in circularization of the oligonucleotide target-specific probe; contacting the first reaction mixture with a nucleic acid amplification assay mixture comprising the first fluorescent molecular probe and the second fluorescent molecular probe; performing a nucleic acid amplification assay such that the target-specific probe bound to the nucleic acid is amplified; measuring a fluorescence ratio; and determining the identity of the nucleic acid sequence based on the fluorescence ratio.

An embodiment relates to the method above, further comprising removing or inactivating from the first reaction mixture oligonucleotide target-specific probes not bound to the nucleic acid sequence.

An embodiment relates to the method above, where the oligonucleotide target-specific probes not bound to the nucleic acid sequence are removed or inactivated via the use of an enzyme.

An embodiment relates to the method above, where at least a one of the plurality of oligonucleotide target-specific probes further comprises one or more copies of a third nucleic acid sequence for binding a third fluorescent molecular probe, and wherein a predetermined ratio of the one or more copies of the first nucleic acid sequence for binding the first fluorescent molecular probe to the one or more copies of the second nucleic acid sequence for binding the second fluorescent molecular probe to the one or more copies of the third nucleic acid sequence for binding the third fluorescent molecular probe is indicative of the distinct nucleic acid sequence the oligonucleotide target-specific probe is configured to bind to.

An embodiment relates to the method above, where the nucleic acid amplification assay is selected from the group consisting of rolling circle amplification, hyperbranched rolling circle amplification, and polymerase chain reaction.

An embodiment relates to a kit for determining the identity of a nucleic acid sequence in a sample comprising the library of oligonucleotide target-specific probes above, and instructions for determining the identity of the nucleic acid sequence based on a measured fluorescence ratio.

An embodiment relates to the kit above, further comprising reagents for conducting a nucleic acid amplification assay.

An embodiment relates to the kit above, further comprising the first fluorescent molecular probe and the second fluorescent molecular probe.

An embodiment relates to the kit above, further comprising an enzyme for removing or inactivating oligonucleotide target-specific probes that do not bind to the target nucleic acid.

An embodiment relates to the kit above, where the reagents for conducting a nucleic acid amplification assay are reagents suitable for conducting a rolling circle amplification assay, a hyperbranched rolling circle amplification assay, or a polymerase chain reaction assay.

**EXAMPLES**

The following describes some embodiments of the current invention more specifically. The general concepts of this invention are not limited to these particular embodiments.

A new ratiometric fluorescence coding concept has been developed that can be employed to significantly expand multiplexing capacity of NAAT by assigning a specific fluorescence color ratio for coding each target. Unlike the common one-color-one-target scheme, a target-specific DNA template with a pre-designed ratio of binding sites for distinctly colored fluorescent probes is produced. The concept has been validated using TSP chemistry coupled with RCA or HRCA reactions. With the custom-designed TSPs targeting infectious disease-related genes, the ratiometric fluorescence coding method for multiplexed detection of 6 nucleic acid targets using only two distinctly colored fluorescent probes, as well as its potential for further expanding the multiplexing capacity has been demonstrated. In addition, an HRCA-based assay has been utilized to detect genomic DNA from a specific STD.

*Example 1*

Ratiometric Fluorescence Coding is described as a new strategy for expanding the multiplexed detection capacity of NAATs by encoding each nucleic acid target with a relative fluorescence ratio. Specifically, in Ratiometric Fluorescence Coding, each target is transformed into a specific DNA template that can hybridize with multiple fluorescently-labeled probes with distinct colors at a pre-designed, unique ratio. In doing so, each target can be detected while obviating the restriction of spectral overlaps between fluorophores. Moreover, since only the relative ratios of fluorescent probe hybridization sites in DNA templates are designed to vary but the sequences of the hybridization sites remain the same, Ratiometric Fluorescence Coding can utilize the same set of fluorescently-labeled probes to detect multiple targets. For demonstrating the concept of Ratiometric Fluorescence Coding, target-specific probe chemistry[20] was utilized for probe design and coupled with rolling circle amplification (RCA), hyperbranched rolling circle amplification (HRCA)[21, 22] or polymerase chain reaction (PCR) for DNA template generation, which allows hybridization with fluorescent-labeled probes. Using the RCA and HRCA assays and only two fluorescencently-labeled probes, the detection of the gene sequences from up to 6 infectious diseases, including 4 sexually transmitted diseases (STDs) and 2 urinary tract infections (UTIs), with distinct and highly resolved fluorescence ratios, is demonstrated below, illustrating that Ratiometric Fluorescence Coding offers ready means for expanding the multiplexed detection capacity of NAATs.

## Design of TSPs for ratiometric fluorescence coding

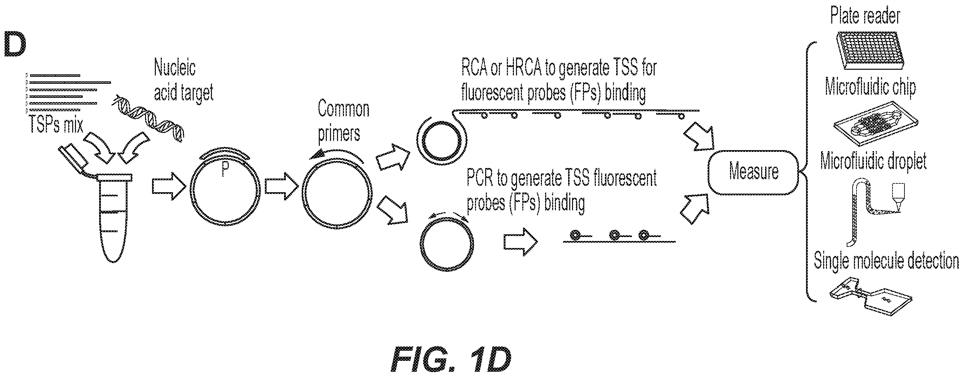

FIGs 1A-1D are schematics providing an overview of Ratiometric Fluorescence Coding for multiplexed nucleic acid detection, according to an embodiment of the invention. Figure 1A shows that the ratiometric fluorescence coding concept utilizes a combination of fluorophore colors (e.g. 2 colors (left) and 3-colors (right) and their fluorescence intensity levels to achieve multiplexed detection. For example, upon hybridization of fluorescent probes with two colors (red and green) onto a single DNA template with a specific ratio, the template-probe complex can be decoded by resolving the intensity level of each color and display the specific red to green (R/G) fluorescence ratio. Figure 1B is an example of target-specific probe (TSP) design for binding with 2-color (green/G and red/R) probes. The internal part of the TSP consists of one or two primer-binding regions common for all the TSPs and several red and green fluorescent probe (FP) binding regions, which have a unique R/G ratio for each TSP. In the presence of corresponding target, TSP is ligated to a closed circle for subsequent amplification. Figure 1C shows six TSPs with different R/G ratios were designed for infectious disease detection. Six circularizable TSPs encoded as R/G ratio of 0:1, 1:2, 1:1, 2:1, 3:1 and 1:0 are designed for targeting gene sequences in *Proteus mirabilis* (PM), *Human immunodeficiency virus 1* (HIV-1), *Neisseria gonorrhoeae* (NG), *Chlamydia trachomatis* (CT), *Treponema pallidum* (TP), and *Escherichia coli* (EC) respectively. Figure 1D shows the ratiometric fluorescence coding assay starts with the mixture of the six TSPs and infectious disease-related DNA target. When binding to a specific target, the phosphorylated 5' end and the 3' end of the TSP are brought into proximity that leads to formation of a circularized probe via ligation. Subsequently, RCA, HRCA or PCR reaction is performed to generate DNA templates with tandem repeats complementary to the TSP sequences, serving as binding sites for fluorophores-labeled probes. Upon hybridization with red and green fluorescent probes, the fluorophores-labeled amplification products are readout in both green and red channels using standard plate reader, microfluidic digital detection or single-molecule detection modalities. The measured R/G fluorescence ratios can be used to identify DNA targets in the initial input.

Ratiometric Fluorescence Coding encodes each target of interest with a specific fluorescence color ratio. This is achieved by transforming each target to a specific DNA template sequence with a pre-designed and unique ratio of binding sites for multiple fluorescently-labeled probes with distinct colors. As such, upon hybridization with fluorescent probes, the template-probe complex displays the specific ratiometric fluorescence code whose intensity of each fluorescence color can be measured to decode and identify the target (Figure 1A). Importantly, in Ratiometric Fluorescence Coding, the number of maximum target-specific fluorescence ratios (i.e., the multiplexing capacity) for a given number of fluorescence colors is governed by a polynomial relationship with the number of fluorescent probe binding sites (S) that can be designed on a DNA template. In the case of 2 fluorescence colors, the maximum achievable fluorescence ratios are $\frac{(S^2+4S-1)}{4}$ for an odd number of S and $\frac{(S^2+3S+3)}{4}$ for an even number of S, respectively. For example, with only a red fluorophore (e.g., AlexaF647) and a green fluorophore (e.g., AlexaF488), up to 7 fluorescence ratios can be achieved if 4 fluorescent probe binding sites (S = 4) are designed on each DNA template, and up to 11 ratios if 5 binding sites (S = 5) are designed on each DNA template. Moreover, in Ratiometric Fluorescence Coding, only the numbers of fluorescence probe binding sites in the DNA templates are designed to vary but the sequences of the fluorescence probe binding sites remain the same. As such, the same fluorescently-labeled probes can be used for detecting multiple targets. These features allow Ratiometric Fluorescence Coding to expand the multiplexing capacity when compared with the traditional one-color-one-target approach and multi-fluorophore strategies without requiring a large number of fluorescently-labeled probes.

TSP chemistry provides an effective means for achieving Ratiometric Fluorescence Coding due to its flexibility for probe design. In this work, each TSP is designed to contain a target-specific hybridization region, a uniquely pre-designed ratio of binding sites for the red and the green fluorescent probes, and general primer sites (Figure 1B). In the presence of its specific DNA target, the initially linear TSP is first hybridized to the target and then ligated to become a circular probe, from which DNA templates can be generated via various amplification techniques to amplify the number of fluorescent probe binding sites at the pre-designed fluorescent probe ratio encoded in each TSP[21, 22, 23]. Following this design principle, six circularizable TSPs were designed to detect 6 common infectious diseases (4 STDs and 2 UTIs) in this work. The target-specific regions of these 6 TSPs target conserved sequences from *Proteus mirabilis* (PM), *Human immunodeficiency virus 1* (HIV-1), *Neisseria gonorrhoeae* (NG), *Chlamydia trachomatis* (CT), *Treponema pallidum* (TP) and *Escherichia coli* (EC). Moreover, the binding sites for the red and the green fluorescent probes were incorporated at specific red to green (R/G) ratios into these TSPs: 1:0 for PM, 1:2 for HIV-1, 1:1 for NG, 2:1 for CT, 3:1 for TP and 1:0 for EC, respectively (Figure 1C).

TSP chemistry has also been coupled with molecular amplification techniques, such as RCA and HRCA, to amplify the fluorescence signals in ratiometric fluorescence coding. More specifically, construction is begun by challenging the infectious disease-related DNA targets to the mixture of the six target-specific, linear TSPs. In the presence of a DNA target, the corresponding linear TSP is hybridized and ligated to form a closed TSP. Then RCA reaction amplifies along the circular TSP to generate DNA templates with hundreds of tandem repeats that serve as hybridization sites for fluorescent probes. In this work, AlexaF488- and AlexaF647-peptide nucleic acid (PNA) as the hybridization probes were employed, because its electro-neutral polypeptide backbones enables high hybridization efficiency with the DNA templates[24, 25]. In addition, the dissociation rate of PNA/DNA hybrid is significantly slower than that of DNA duplexes, thus allowing the removal of non-hybridized, excess PNA from the reaction without reducing the fluorescence signals of the template-probe complexes due to dissociation of hybridized fluorescent probes. After fluorescent PNA probe hybridization and purification, the red and the green fluorescence intensities of the template-probe complexes are measured, and the resultant R/G fluorescence ratio is used to identify the DNA target (Figure 1D).

*Example 2*

**Ratiometric fluorescence detection with RCA in a bulk assay using a plate reader**

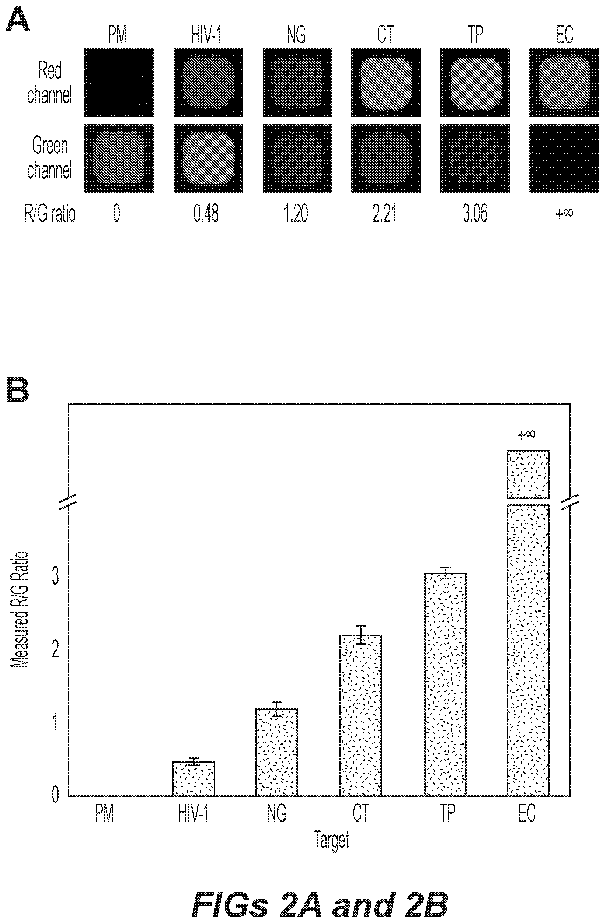

FIGs 2A and 2B show results of six independent experiments for detecting the six infectious disease-related synthetic targets, according to an embodiment of the invention. Figure 2A shows RCA-based ratiometric fluorescence coding assay was validated via detecting six infectious disease-related genes. With PM, HIV-1, NG, CT, TP and EC synthetic targets, the resultant fluorophores-labeled RCA products were scanned under both red (top row) and green (bottom row) channels. From left to right in each channel, the fluorescent spots represent fluorophores-labeled RCA products resultant from PM, HIV-1, NG, CT, TP and EC synthetic targets, respectively. Moreover, measured R/G ratio for each sample is close to the pre-designed ratio. The spots of fluorophores-labeled PM amplification products present fluorescence only in green channel with a measured R/G ratio of 0. The spots of HIV-1 sample show weak fluorescence in red channel and strong fluorescence in green channel with a measured R/G ratio of 0.48. The spots of NG sample show nearly equivalent fluorescence intensities in red and green channel with a measured R/G ratio of 1.20. In contrast, the spots of CT and TP samples show strong fluorescence in red channel and weak fluorescence in green channel with measured R/G ratios of 2.21 and 3.06 respectively. And the spots of EC sample show fluorescence only in the red channel with a measured R/G ratio of ∞. Figure 2B shows that the assay was replicated in six independent experiments for detecting the six infectious disease-related synthetic targets. The small error bars of measured R/G ratios indicate a robust reproducibility.

Using the RCA-based ratiometric fluorescence coding assay, the detection of six infectious disease-related gene sequences with distinct fluorescence ratios was first demonstrated. To do so, six synthetic oligonucleotides were derived from conserved gene sequences of PM, HIV-1, NG, CT, TP and EC as the targets, and challenged with a mix of six TSPs, the red and the green fluorescence intensities of each sample at the end of the RCA reaction and probe hybridization was measured. Fluorescent images of the six samples reveal that the six targets indeed yielded fluorescence ratios that were noticeably different from each other (Figure 2A). Moreover, the measured R/G ratio of each target closely matched its designed R/G ratio. For example, for the PM sample, the corresponding TSP of which is inserted with only green fluorescent probe binding sites, only green fluorescence and no red fluorescence was detected, resulting in a measured R/G ratio of 0. While for the HIV-1 sample weak red fluorescence and strong green fluorescence was observed and a R/G ratio of ~0.5 was measured. In contrast, for the TP sample, strong red fluorescence and weak green fluorescence was observed, and a R/G ratio of ~3.0 was measured. Whereas for the EC sample, the TSP of which contains only red fluorescent probe sequence, only fluorescence in the red channel was observed and thus the R/G ratio was measured to be infinity. These fluorescence detection results clearly indicate that the RCA-based ratiometric fluorescence coding assay can achieve multiplexed detection of nucleic acid targets based on distinct R/G ratios.

The RCA-based ratiometric assay detects the six infectious disease-related gene sequences with high reproducibility. Here, the assay for PM, HIV-1, NG, CT, TP and EC synthetic targets was replicated in four separate experiments, and the average R/G ratios were plotted with error bars based on the four independent measurements (Figure 2B). The close match between the measured R/G ratios and the pre-designed R/G ratios provides strong support for the design principle of ratiometric fluorescence coding. In addition, the small error bars of measured ratios indicate that each target-specific ratio is indeed significantly different from each other and that the assay is highly reproducible. Taken together, these results also suggest that additional R/G ratios can be incorporated into the RCA-based assay by inserting additional binding sites in the TSPs, which can expand the multiplexed detection capacity of the assay.

*Example 3*

**Ratiometric fluorescence detection with HRCA in a bulk assay using a plate reader**

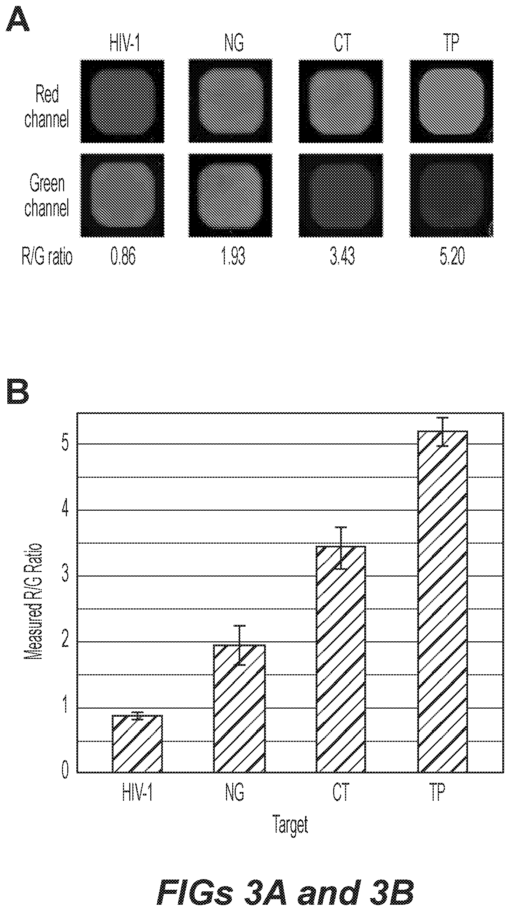

FIGs 3A and 3B show results of an assay detecting four infectious disease-related synthetic targets, according to an embodiment of the invention. Figure 3A shows HRCA-based ratiometric fluorescence coding assay used to verified using the four TSPs and STD synthetic targets. In the presence of HIV-1, NG, CT and TP synthetic targets, HRCA generates dsDNA amplification products that are hybridized with red and green fluorescent PNA probes. The resultant fluorophores-labeled HRCA products were scanned under Typhoon image scanner in both red (top row) and green (bottom row) channels. From left to right in each channel, the fluorescent spots represent fluorophores-labeled HRCA products of HIV-1, NG, CT and TP samples respectively. The spots of fluorophores-labelled HIV-1 amplification products show weak fluorescence in red channel and strong fluorescence in green channel with measured R/G ratios of 0.86. While the spots of NG, CT and TP samples show strong fluorescence in red channel and weak fluorescence in green channel with measured R/G ratios of 1.93, 3.43 and 5.20 respectively. Figure 3B shows the HRCA-based assay was also replicated in four separated experiments. Each measured target-specific R/G ratio is significantly different from each other and their small error bars indicate high reproducibility of the HRCA-based assay.

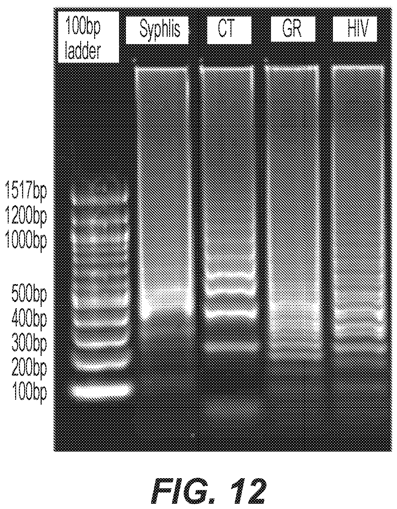

Toward improving the sensitivity of multiplexed detection via ratiometric fluorescence coding, we show the compatibility between the instant method and HRCA. Compared with the linear amplification mechanism in RCA, HRCA employs a second primer to achieve exponential amplification of double-stranded DNA (dsDNA) templates (Figure 12) for fluorescent probes [26, 27]. The PNA probe plays a critical role here, because strong binding affinity between PNA and DNA ensures the PNA probes can hybridize to the DNA templates even in the presence of complementary DNA strands. To verify the HRCA-based assay, four TSPs were mixed with each of the HIV-1, NGNG, CT and TP synthetic targets and detected the red and the green fluorescence intensities of each sample at the end of the HRCA reaction and probe hybridization. Similar to the results from RCA reactions, fluorescent images of these four HRCA samples also show fluorescence ratios that were noticeably distinct (Figure 3A). For example, relatively weak red fluorescence and strong green fluorescence for the HIV-1 sample were observed, yielding a measured R/G ratio of ~0.8. On the other hand, strong red fluorescence and weak green fluorescence could be observed from the CT sample and the TP sample, with measured R/G ratios of ~3.4 and ~5.2, respectively. Similarly, the experiments were replicated four times and plotted the measured R/G ratios with errors bars (Figure 3B). The results indicated high reproducibility of the HRCA-based assay for detecting STD-related gene sequences based on distinct R/G fluorescence ratios.

Having verified robust multiplex detection through both HRCA-based and RCA-based ratiometric fluorescence coding assays, it is important to note that the measured R/G ratio for each HRCA sample was higher than that for its RCA counterpart. The difference in measured R/G ratios may be caused by the difference in self-quenching of neighboring Alexa Fluro 647 molecules between the HRCA and the RCA samples [28, 29]. Specifically, in RCA, the template-probe complex that is formed when thousands of probes hybridize onto long single-stranded DNA templates could fold into a spherical conformation in solution[30, 31]. This conformation confines the Alexa Fluro 647 molecules, and their proximity causes them to quench each other, thus reducing the red fluorescence intensity in RCA samples. In contrast, double strand DNA templates produced from HRCA were only hundreds of base pairs in length (Figure 12), which allowed much fewer fluorescent probes to hybridize onto each template and reduced the potential for self-quenching of neighboring Alexa Fluro 647 molecules. As a result, the intensities of red fluorescence in HRCA samples were elevated, resulting in higher R/G ratios.

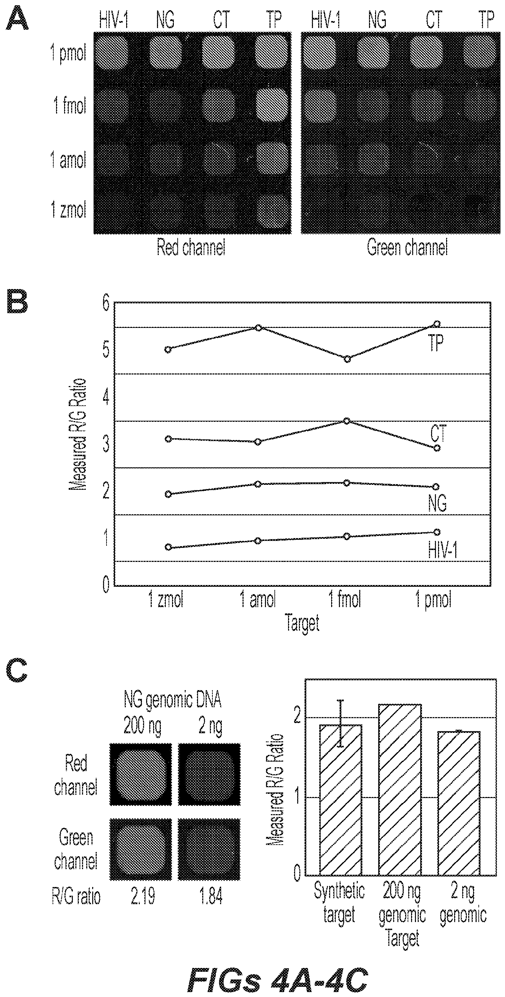

Additionally, the sensitivity of the HRCA-based ratiometric fluorescence coding assay and its ability to detect genomic DNA was evaluated. Here, four STD synthetic targets were titrated to 1 pmol, 1 fmol, 1 amol to 1 zmol, tested with the HRCA-based assay, and their red and green fluorescence intensities were measured. For each target, as the input concentration lowered, both the red and the green fluorescence intensities expectedly reduced as a result of lower concentration of dsDNA templates generated from lower concentration of target, though all four targets were still detected at 1 zmol (Figure 4A). More importantly, for each target, even as the input concentration decreased by 9 orders of magnitude, the measured R/G ratios remained fairly consistent (Figure 4B). For example, the R/G rations for NG synthetic targets remained at ~2 for the 4 input target concentrations. The consistency of measured R/G ratios for the 4 targets across a wide range of input concentrations further illustrates the robustness of the ratiometric fluorescence coding method. Finally, the HRCA-based assay was used to detect 200 ng and 2 ng NG genomic DNA (extracted from *Neisseria gonorrhoeae* Strain FA1090, ATCC). Strong red and green fluorescence was observed in the sample with 200 ng NG genomic DNA sample and noticeably weaker fluorescence in the sample with 2 ng NG genomic DNA (Figure 4C, left). Moreover, the measured R/G ratios in both genomic DNA samples were comparable to that of the NG synthetic target (Figure 4C, right). These results therefore suggest that the HRCA-based ratiometric fluorescence coding assay can also detect STD genomic DNA based on its pre-designed fluorescence ratio.

FIGs 4A - 4C show results of various assays testing the sensitivity of a ratiometric fluorescence coding method according to an embodiment of the invention. Figure 4A shows the sensitivity of the HRCA-based ratiometric fluorescence coding method was verified using a gradient input of each STD synthetic targets ranging from 1 pmol, 1 fmol, 1 amol to 1 zmol. The fluorophore-labeled HRCA samples were scanned under Typhoon scanner in both red (left panel) and green (right panel) channels. The synthetic target inputs are HIV-1, NG, CT and TP from left to right in each row, and target input concentrations are 1 pmol, 1 fmol, 1 amol and 1 zmol from top to bottom in each column. The fluorescence intensities in both red and green channels were observed to decline as target input decreasing due to the deduction of dsDNA templates generated through HRCA. Figure 4B shows that for each target, the measured R/G ratios remained fairly consistent across the input concentrations, further indicating the robustness of the method. In Figure 4C, the HRCA-based assay was performed to detect 200 ng and 2ng of NG genomic DNA. The resultant fluorophores-labeled HRCA samples were scanned under in both red and green channels, and R/G fluorescence ratios were measured (left). R/G ratios of two NG genomic DNA samples were measured to be comparable to that of NG synthetic target using the HRCA-based assay (right).

*Example 4*

**Ratiometric fluorescence detection with PCR or HRCA in a digital assay using microfluidic array chip**

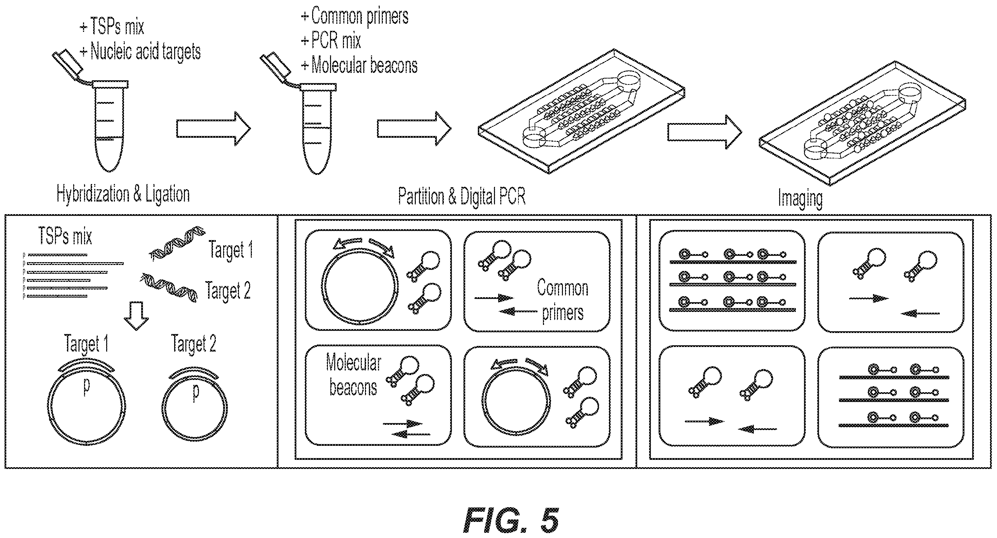

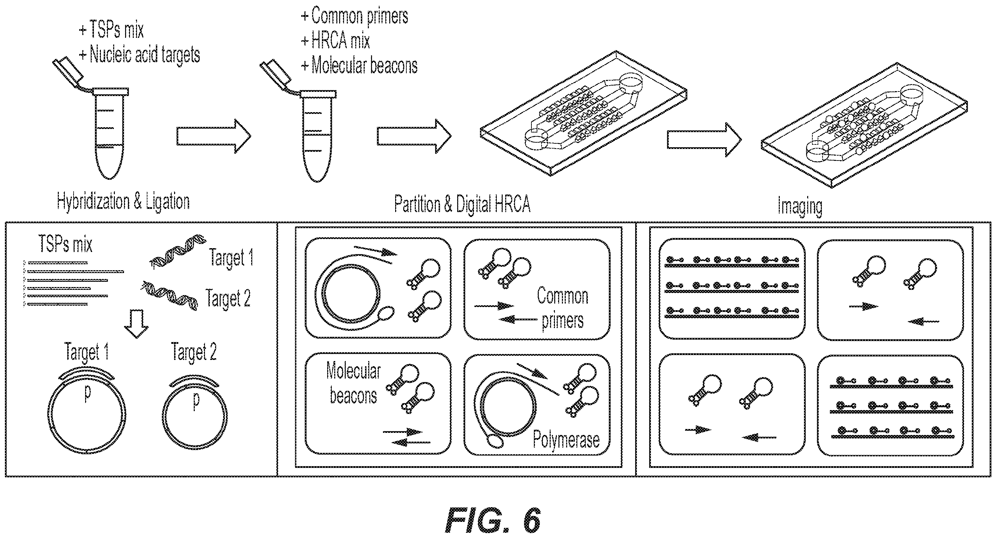

Example of coupling ratiometric fluorescence coding with PCR or HRCA in a digital assay using microfluidic chip (Figure 5 and 6). The assay starts with the mixture of custom-designed TSPs and nucleic acid targets of interest. In the presence of specific target, the linear TSP is ligated to become circularized probe. Subsequently, samples are loaded onto a microfluidic digital chip where single copy of TSP is partitioned into each well for subsequent digital PCR or HRCA. The PCR or HRCA reaction generates DNA templates with tandem repeats complementary to the TSP sequences, serving as binding sites for molecular beacons. Upon hybridization with molecular beacons, the fluorophores-labeled amplification products in each digital well on the chip are measured and the resultant fluorescence color ratio is used to identify the nucleic acid target.

FIG. 5 is a schematic showing a digital microfluidic platform for Ratiometric Fluorescence coding assay using PCR, according to an embodiment of the invention. The assay starts with the mixture of TSPs and nucleic acid targets. The internal part of TSP consists of two primer-binding regions common for all the TSPs and several red and green molecular beacon-binding regions, which have a unique R/G ratio for each TSP (target). When binding to a specific target, the phosphorylated 5' end and the 3' end of the TSP are brought into proximity that leads to formation of a circularized probe via ligation. Subsequently, the mixture of circularized TSPs, PCR reaction mixture and differently labeled molecular beacons are loaded onto the microfluidic chip and each well on the chip contains either 0 to 1 copy of circularized TSP. PCR is performed in each well to generate DNA templates using the circularized TSP as template and two common primers. The resultant DNA templates from PCR amplification serve as binding sites for distinctly labeled molecular beacons. Upon hybridization with red and green molecular beacons, the fluorescence intensities of distinct colors from each digital well on the chip are measured and the resultant fluorescence color ratio is used to identify the nucleic acid target.

FIG. 6 is a schematic showing a digital microfluidic platform for Ratiometric Fluorescence coding assay using HRCA, according to an embodiment of the invention. The assay starts with the mixture of TSPs and nucleic acid targets. The internal part of TSP consists of two primer-binding regions common for all the TSPs and several red and green molecular beacon-binding regions, which have a unique R/G ratio for each TSP (target). When binding to a specific target, the phosphorylated 5' end and the 3' end of the TSP are brought into proximity that leads to formation of a circularized probe via ligation. Subsequently, the mixture of circularized TSPs, HRCA reaction mixture and differently labeled molecular beacons are loaded onto the microfluidic chip and each well on the chip contains either 0 to 1 copy of circularized TSP. HRCA is performed in each well to generate DNA templates using the circularized TSP as template and two common primers. The resultant DNA templates from HRCA serve as binding sites for distinctly labeled molecular beacons. Upon hybridization with red and green molecular beacons, the fluorescence intensities of distinct colors from each digital well on the chip are measured and the resultant fluorescence color ratio is used to identify the nucleic acid target.

*Example 5*

**Ratiometric fluorescence detection with PCR or HRCA in a digital assay using microfluidic droplets**

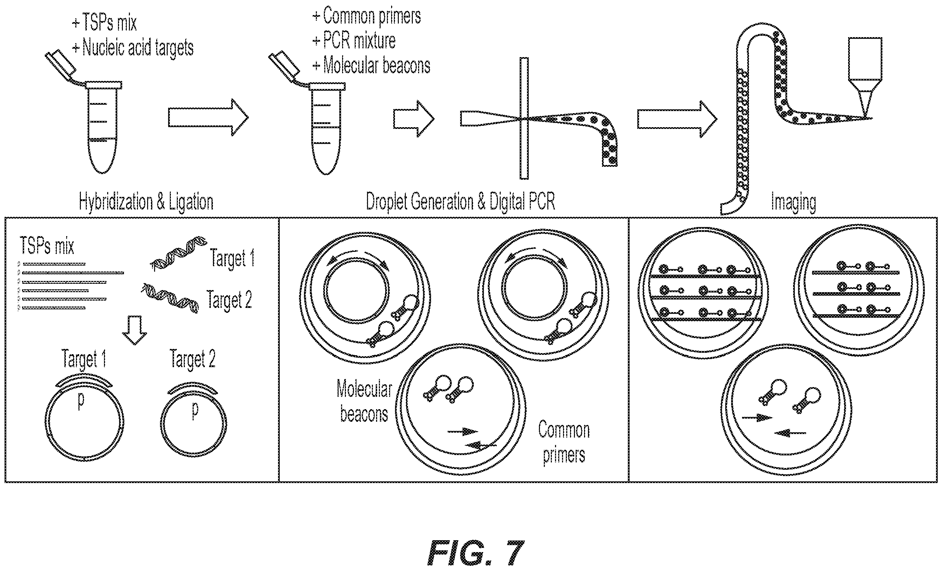

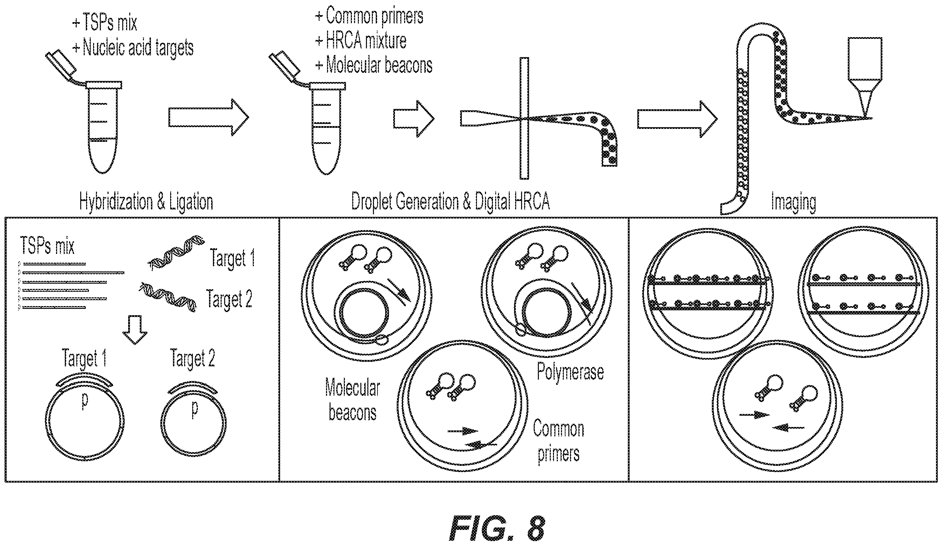

This is an example of coupling ratiometric fluorescence coding with PCR or HRCA in a digital assay using microfluidic droplet platform (Figure 7 and 8). The assay starts with the mixture of custom-designed TSPs and DNA targets of interest. In the presence of specific target, the linear TSP is ligated to become circularized probe. Samples are then loaded into digital droplets using a microfluidic droplet chip and single copy of TSP is encapsulated into each droplet for subsequent digital PCR or HRCA. The PCR or HRCA amplification generates DNA templates with tandem repeats complementary to the TSP sequences, serving as binding sites for molecular beacons. Upon hybridization with molecular beacons, the fluorescence signals of each droplet are measured in a sequential manner via a custom-built, multi-color confocal fluorescence spectroscopic (CFS) instrument and the resultant fluorescence color ratio is used to identify the nucleic acid target.

FIG. 7 is a schematic showing a digital droplet platform for Ratiometric Fluorescence coding assay using PCR, according to an embodiment of the invention. The assay starts with the mixture of TSPs and nucleic acid targets. The internal part of TSP consists of two primer-binding regions common for all the TSPs and several red and green molecular beacon-binding regions, which have a unique R/G ratio for each TSP (target). When binding to a specific target, the phosphorylated 5' end and the 3' end of the TSP are brought into proximity that leads to formation of a circularized probe via ligation. Subsequently, single copy of TSP is encapsulated into droplet via droplet generation and digital PCR is performed to generate DNA templates using the circularized TSP as template within each droplet. The resultant DNA templates from PCR amplification serve as binding sites for the distinctly-labeled molecular beacons. Upon molecular beacon hybridization, fluorescence detection of each droplet is performed in a sequential manner via a custom-built, multi-color confocal fluorescence spectroscopic (CFS) instrument. The fluorescence intensities of distinct colors in each droplet is measured and the resultant fluorescence ratio is used to identify the nucleic acid target.

FIG. 8 is a schematic showing a digital droplet platform for Ratiometric Fluorescence coding assay using HRCA, according to an embodiment of the invention. The assay starts with the mixture of TSPs and nucleic acid targets. The internal part of TSP consists of two primer-binding regions common for all the TSPs and several red and green molecular beacon-binding regions, which have a unique R/G ratio for each TSP (target). When binding to a specific target, the phosphorylated 5' end and the 3' end of the TSP are brought into proximity that leads to formation of a circularized probe via ligation. Subsequently, single copy of TSP is encapsulated into droplet via droplet generation and digital HRCA is performed to generate DNA templates using the circularized TSP as template within each droplet. The resultant DNA templates from PCR amplification serve as binding sites for the distinctly-labeled molecular beacons. Upon molecular beacon hybridization, fluorescence detection of each droplet is performed in a sequential manner via a custom-built, multi-color confocal fluorescence spectroscopic (CFS) instrument. The fluorescence intensities of distinct colors in each droplet is measured and the resultant fluorescence ratio is used to identify the nucleic acid target.

*Example 6*

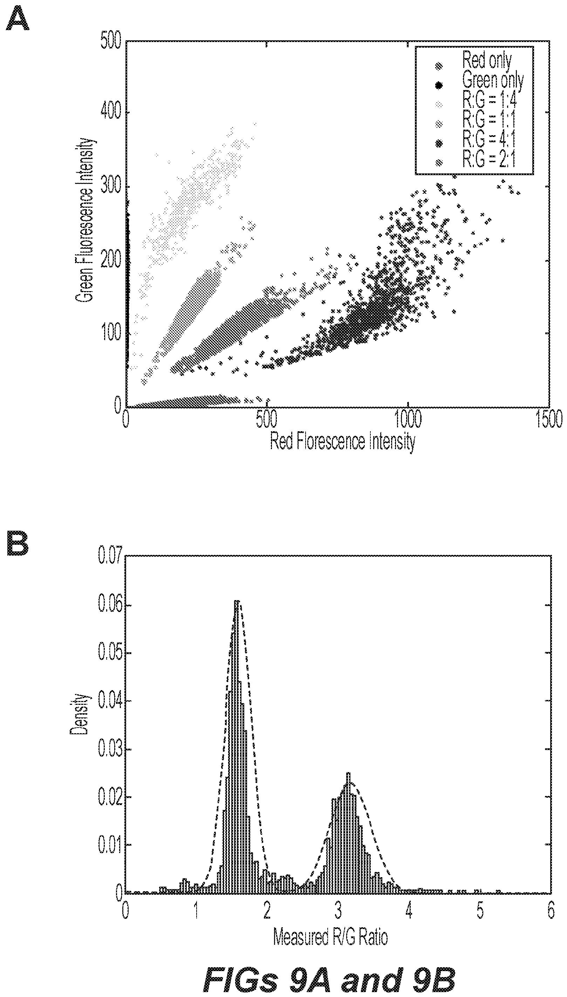

The ratiometric fluorescence coding coupled with HRCA in digital droplets has been verified for multiplexed detection of using six custom-designed TSPs targeting six common STDs. The target-specific regions of these 6 TSPs target conserved sequences from *Microplasma genitalium* (MG), *Herpes simplex virus* (HSV), *Neisseria gonorrhoeae* (NR), *Human immunodeficiency virus* (HIV), *Treponema pallidum* (TP) and *Chlamydia trachomatis* (CT). We also incorporated the binding regions for the red and the green fluorescent probes at specific red to green (R/G) ratios into these TSPs: 0:1 for GM, 1:4 for HSV, 1:1 for NR, 2:1 for HIV-1, 4:1 for TP and 1:0 for CT, respectively. We used six synthetic oligonucleotides derived from conserved gene sequences of MG, HSV, NG, HIV, TP and CT as the targets, challenged each of them to the mix of TSPs, and measured the red and the green fluorescence intensities of droplets from each sample. After fluorescence measurement on the confocal microscope system, samples with different DNA targets indeed yielded R/G fluorescence ratios that were noticeably distinct from each other (Figure 9). The scatter plot for red and green fluorescence intensities in each sample has revealed that the six target-specific R/G fluorescence ratios were unambiguously separated (Figure 9A). In addition, we have demonstrated the Digital Ratiometric Fluorescence Coding method for multiplexed detection of different STD-related gene sequences in a single reaction. We added both NR and HIV synthetic targets into initial hybridization and ligation reaction containing the mix of six TSPs. After droplet generation and amplification, we measured the red and green fluorescence intensities of each droplet. The resultant R/G ratios present two populations that are unambiguously separated via double Gaussian distribution fit (Figure 9B).

FIGs 9A and 9B are data graphs showing that the digital droplet platform for Ratiometric Fluorescence coding assay using HRCA has been verified using six TSPs and STD synthetic targets, according to an embodiment of the invention. (Figure 9A) The scatter plot for red and green fluorescence intensities in each sample reveals that the six target-specific R/G fluorescence ratios are noticeably distinct and can be unambiguously separated. (Figure 9B) We have demonstrated the Digital Ratiometric Fluorescence Coding method for multiplexed detection of different STD-related gene sequences in a single reaction. We added both NR and HIV synthetic targets into initial hybridization and ligation reaction containing the mix of six TSPs. After droplet generation and amplification, we measured the red and green fluorescence intensities of each droplet. The resultant R/G ratios present two populations that are unambiguously separated via double Gaussian distribution fit.

*Example 7*

**Ratiometric fluorescence detection with RCA in a single molecule assay using confocal fluorescence spectroscopy such as cylindrical illumination confocal spectroscopy** (CICS)

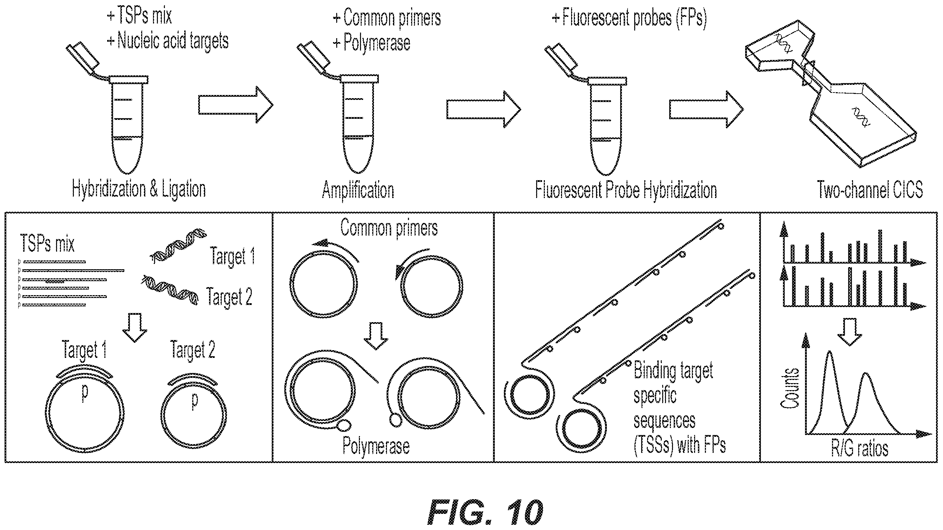

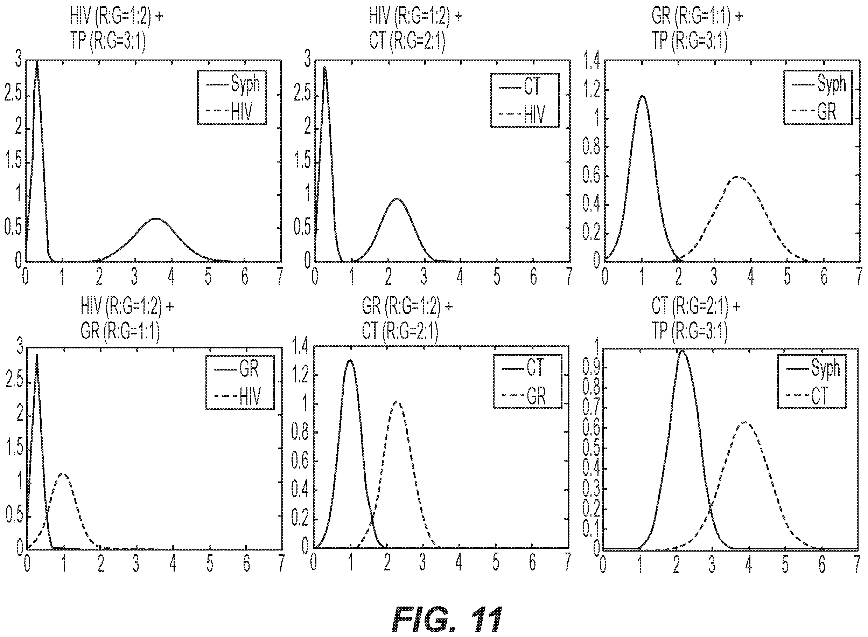

Example of coupling the ratiometric fluorescence coding with RCA using the cylindrical illumination confocal spectroscopy (CICS) (Figure 10). The assay starts with the mixture of custom-designed TSPs and DNA targets. In the presence of specific targets, the linear TSP is ligated to become a circularized probe. Subsequently, RCA reaction is performed to generate DNA templates with tandem repeats complementary to the TSP sequences, serving as binding sites for fluorophores-labeled PNA probes. Upon hybridization with PNA-AF647 (red) and PNA-AF488 (green), the template-probe complex is flowed through a microchannel and excited by lasers through an objective as it transits the observation volume (OV). The resultant coincident peaks in red and green channels are then be processed to yield the R/G ratios distributions to identify nucleic acid targets in the initial input. The ratiometric fluorescence coding coupled with RCA using single molecule detection has been validated via the four TSPs targeting STD sequences and corresponding nucleic acid targets. With different STD DNA targets as input, the resultant R/G ratios distributions are noticeably distinct (Figure 11). For example, for the sample with HIV-1 (designed R/G ratio of 0.5) and TP (designed R/G ratio of 3) targets, we observed two distinct R/G ratio populations, of which one centered at ~0.4 and other one centered at ~3.6. While sample with HIV-1 and CT (designed R/G ratio of 32) targets yields two measured R/G ratio populations centering at ~0.4 and ~2.2 respectively.

Also in Figure 10, the single-molecule assay starts with the mixture of TSPs and nucleic acid targets. The internal part of TSP consists of one or two primer-binding regions common for all the TSPs and several red and green fluorescent probe-binding regions, which have a unique R/G ratio for each TSP (target). When binding to a specific target, the phosphorylated 5' end and the 3' end of the TSP are brought into proximity that leads to formation of a circularized probe via ligation. Subsequently, amplification reaction is performed to generate long DNA templates with thousands of tandem repeats complementary to the TSP sequences, serving as binding sites for fluorescent probes. Upon hybridization with red and green fluorescent probes, the template-probe complex is flowed through the a microchannel and excited by lasers through an objective as it transits the observation volume (OV). The resultant coincident peaks in red and green channels are then be processed to yield the R/G ratios distributions to identify nucleic acid targets in the initial input.

FIG. 11 is a series of data graphs showing that the single-molecule Ratiometric Fluorescence Coding assay was verified using four TSPs and STD synthetic targets, according to an embodiment of the invention. In the presence of HIV-1, NG, CT and TP synthetic targets, RCA generates ssDNA amplification products that are hybridized with red and green fluorescent PNA probes. The resultant fluorophores-labeled RCA products were flowed through the a microchannel and excited by lasers on two-channel CICS. The fluorescence intensities of distinct colors on each fluorophores-labeled RCA molecule were measured and the resultant R/G ratios distributions were used to identify nucleic acid targets in the initial input.

*Example 8*

Future experiments to improve the method for highly multiplex and sensitive detection of nucleic acids follow. First, the multiplexing capacity of the method can be readily expanded by using more fluorescence colors and designing more fluorescent probe binding sites. For example, using 3 colors and 5 binding sites (S=4), the maximum number of ratio can reach 40 (Table 2). In addition, while the current interaction of Ratiometric Fluorescence Coding has been demonstrated for identifying one potential target out of multiple possible candidates, its utility can be extended to simultaneously detecting multiple targets in a single reaction by coupling it with single molecule detection method [32, 33]. The molecule-by-molecule measurement scheme in single molecule detection system can measure fluorescently-labeled template-probe complexes one at a time in both red and green channel simultaneously, thus allows differentiation of template-probe complexes from different targets based on their distinct R/G ratios, which can be utilized for simultaneous detection of multiple targets in a single reaction. Therefore, given the good performance of the ratiometric fluorescence coding method and the means to improve it, the method has the potential to perform large-scale multiplexed detection of nucleic acids upon further development.

*Example 9*

**Experimental Section**

Materials and reagents

All DNA oligonucleotides including synthetic targets, target-specific probes, and primers were purchased from Integrated DNA Technologies, Inc. (Coralville, IA) and both fluorescently-labeled peptide nucleic acid (PNA) probes were purchased from PNA Bio (Newbury Park, CA). Reagents for ligation, RCA, and HRCA reactions, including 9°N™ DNA Ligase, 10× 9°N™ DNA Ligase Reaction Buffer, Phage T4 Gene-32 Protein, Phage ø29 DNA Polymerase, Exonuclease I (*E. coli*), Exonuclease III (*E. coli*), 10× Isothermal Reaction Buffer, Bst DNA polymerase, and 10× Bst Polymerase Reaction Buffer were purchased from New England BioLabs, Inc. (Ipswich, MA). Deoxyribonucleotide triphosphate (dNTP) was purchased from Invitrogen Corp. (Carlsbad, CA). Formamide, sodium chloride (NaCl), EDTA (pH 8.0), Bovine Serum Albumin (BSA), Betaine and $H_2O$ (PCR grade) were purchased from Sigma-Aldrich Corp. (St. Louis, MO).

Nucleic acid sequences

Target-specific probes targeting gene sequences in *Proteus mirabilis* (PM, 238:275 from AF240693) *Human immunodeficiency virus 1* (HIV-1, 258:305 from KC966998), *Neisseria gonorrhoeae* (NG, 362:411 from X52364), *Chlamydia trachomatis* (CT, 50:98 from JX648604), *Treponema pallidum* (TP, 801:846 from KC966998), and *Escherichia coli* (EC, 189:230 from AY447194) were designed in-house. All target-specific probe and synthetic target sequences are given in Table 1. The sequence of the RCA primer is 5'-CTAAAGCTGAGACATGACGAGTC and the sequence of the additional primer for performing HRCA is 5'- TCAGAACTCACCTGTTAG. The sequences of the red and the green PNA probes are [5'AlexaF647] TCAGAACTCACCTGTTAG and [5'AlexaF488] CCCTAACCCTAACCCTAA, respectively.

Target-specific probe hybridization and ligation

Our Ratiometric Fluorescence Coding assays began with target-specific probe hybridization and ligation, which were achieved in a single-tube reaction. The 25-μL reaction mixture contained 45 nM of each linear target-specific probe, synthetic DNA targets (at different concentrations), 0.4 Units/μL 9°N™ DNA Ligase, and 1× 9N DNA Ligase Reaction Buffer (10 mM Tris-HCl, 600 μM ATP, 2.5 mM $MgCl_2$, 2.5 mM Dithiothreitol, 0.1% Triton X-100, pH 7.5 at 25°C). Target-specific probe hybridization and ligation was performed at 60 °C for 1 hour. For subsequent HRCA reaction, only 100 pM of each target-specific probe were ligated in the presence of synthetic DNA targets or pathogen genomic DNA to reduce non-specific amplification and improve signal-to-noise-ratio.

RCA and HRCA reactions

RCA or HRCA were performed immediately after ligation of target-specific probes to generate DNA templates that allow hybridization with fluorescently-labeled probes. For RCA, 2 μL of the ligation product was mixed with 800 nM rolling-circle primer, 400 μM dNTPs, 10 U/μL Phage ø29 DNA polymerase, and 1× Isothermal Reaction Buffer (50 mM Tris•HCl (pH 7.5), 10 mM $MgCl_2$, 200 μg/mL acetylated BSA) to a final volume of 25 μL. Of note, due to its high strand-displacing activity, Phage ø29 DNA polymerase was added last to the reaction mixture, which was then immediately incubated at 31 °C for 1 hour to perform RCA, followed by 70 °C for 10 minutes to inactivate the polymerase and stop the reaction.

For HRCA, 25 μL of the ligation product was first treated with 10 units of Exonuclease I and 50 units of Exonuclease III at 37 °C for 1 hour to remove extra linear target-specific probes that were not circularized, followed by a brief incubation at 85 °C for 10 minutes to inactivate the enzymes. Then 4 μL of this enzyme-treated ligation product was mixed with 400 nM of each of the two rolling-circle primers, 400 μM dNTPs, 40 ng/ml Phage T4 Gene-32 Protein, 10 U/μL Bst DNA polymerase, and 1× Bst Polymerase Reaction Buffer (20 mM Tris•HCl (pH 8.8), 10 mM KCl, 2.7 mM $MgSO_4$, 5% v/v DMSO, 0.1% Triton × 100) to a final volume of 25 μL. The reaction mixture was incubated at 60 °C for 1 hour to perform HRCA, followed by 85 °C for 10 minutes to inactivate the polymerase and stop the reaction.

Fluorescent PNA hybridization and detection

10 μL RCA or HRCA product was mixed with 500 nM AlexaF488-labeled PNA probe and 500 nM AlexaF647-labeled PNA probe in a PNA hybridization buffer (40% Formamide, 10 mM NaCl, and 50 mM Tris•HCl (pH 8)), heated at 85 °C for 5 minutes, and then incubated at room temperature in the dark for 2 hours to allow hybridization. Non-hybridized, excess fluorescent PNA probes were removed by using S400-HR microspin columns (GE Healthcare). Each purified sample was pipetted into a well of a 384-well plate (Corning Inc.) and read out on a Typhoon image scanner (GE Healthcare) in both the red channel (peak excitation at 633 nm with 670 nm band-pass emission filter) and the green channel (peak excitation at 488 nm with 526 nm short-pass emission filter) at a photomultiplier tube (PMT) voltage of either 600 V or 650 V. Of note, for each experiment, the PMT voltage was kept the same between the red channel and the green channel (i.e., both at 600 V or both at 650 V). Between experiments, however, the PMT voltage was sometimes adjusted between either at 600 V or at 650 V.

Data analysis

Red and green fluorescence intensities of each sample measured by the Typhoon image scanner were quantified by ImageQuantTL software (GE Healthcare). The R/G ratio was calculated via dividing the red fluorescence intensity by the green fluorescence intensity.

## Table 1

| Padlock probes | Sequence |
|---|---|
| Padlock_PM | /5Phos/GACCAGTTTTGCTTGCGCCCCCTAACCCTAACCCTAAGACTCGTCATGTCTCAGCTTTAGCCCTAACCCTAA CCCTAACGTGCTGCACGTTTAGCAC |
| Padlock_TP | /5Phos/CTGTCGCACCGTGAGTTCATCTCAGAACTCACCTGTTAGGACTCGTCATGTCTCAGCTTTAGCCCTAACCCT AACCCTAATTTTTCAGAACTCACCTGTTAGTTTTTCAGAACTCACCTGTTAGAAAGGGCTGATGGCTCTGAG |
| Padlock_CT | /5Phos/CCTTATGATCGACGGAATTCTGTGTCAGAACTCACCTGTTAGGACTCGTCATGTCTCAGCTTTAGCCCTAAC CCTAACCCTAATTTTTCAGAACTCACCTGTTAGGGGAATCCTGCTGAACCAAG |
| Padlock_GR | /5Phos/GGAACCCGATATAATCCGCCCTTTCAGAACTCACCTGTTAGGACTCGTCATGTCTCAGCTTTAGCCCTAACC CTAACCCTAAATATTGTGTTGAAACACCGCCC |
| Padlock_HIV | /5Phos/CCAAATGAGAGAACCAAGGGGAAGGACTCGTCATGTCTCAGCTTTAGCCCTAACCCTAACCCTAATTTTTC AGAACTCACCTGTTAGTTTTCCCTAACCCTAACCCTAACAGGGCCTATTGCACCAGG |
| Padlock_EC | /5Phos/GGCGTGGTGTAGAGCATTACGTCAGAACTCACCTGTTAGGACTCGTCATGTCTCAGCTTTAGTCAGAACTC ACCTGTTAGATATCGTCCACCCAGGTGTTC |

| Synthetic targets | Sequence |
|---|---|
| Target_PM | 5'-GGCGCAAGCAAAACTGGTCGTGCTAAACGTGCAGCACG |
| Target_TP | 5'-TAGTCGATGAACTCACGGTGCGACAGCTCAGAGCCATCAGCCCTTTTCAGC |
| Target_CT | 5'-CACAGAATTCCGTCGATCATAAGGCTTGGTTCAGCAGGATTCCCCACAG |
| Target_GR | 5'-AAGGGCGGATTATATCGGGTTCCGGGCGGTGTTTCAACACAATATGGCGG |
| Target_HIV | 5'-TGTCACTTCCCCTTGGTTCTCTCATTTGGCCTGGTGCAATAGGCCCTGCATGC |
| Target_EC | 5'-CG TAATGCTCTA CACCACGCCG AACACCTGGG TGGACGATAT |

## Table 2

| Number of colors | Number of binding sites (S) | | | | |
|---|---|---|---|---|---|
| | 4 | 5 | 6 | ... | 10 |
| 2 | 7 | 11 | 13 | | 41 |
| 3 | 22 | 40 | 55 | | 226 |
| 4 | 51 | 103 | 161 | | 849 |

*Example 10*

**Use of pair of probes to bind to and detect the identity of a nucleic acid sequence.**

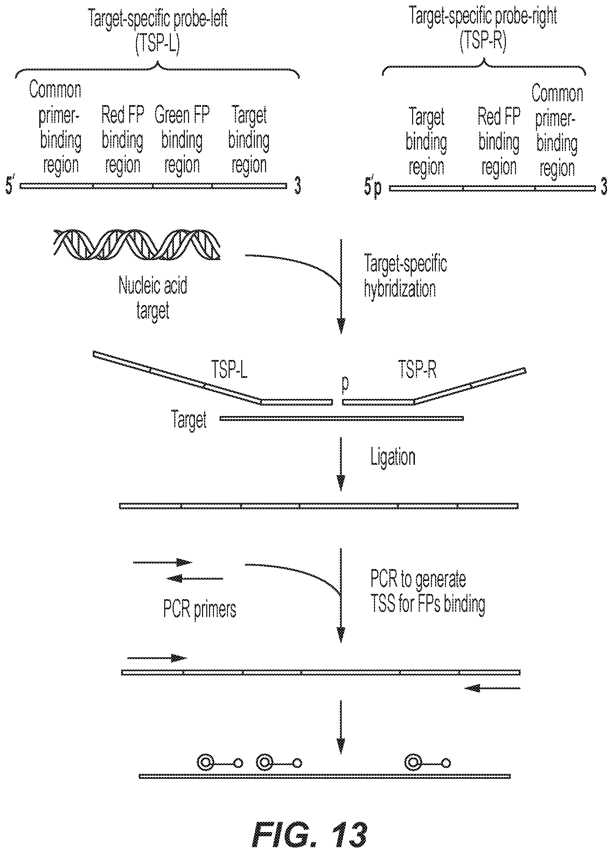

In an alternative embodiment, a pair of target-specific probes are used to bind to and detect the identity of a nucleic acid sequence. Figure 13 shows an alternative design of target-specific probes (TSPs) for achieving Ratiometric Fluorescence Coding. For each nucleic acid target of interest, one pair of TSPs are designed consisting of two oligonucleotides (target-specific probe-left (TSP-L) and target-specific probe-right (TSP-R)) that recognize adjacent target sites on the nucleic acid sequences. TSP-L contains a target-specific hybridization region, binding sites for red and green fluorescent probes and the sequence recognized by the PCR forward primer. TSP-R is a 5' end- phosphorylated oligonucleotide containing a target-specific hybridization region, binding sites for red and green fluorescent probes and the sequence recognized by the PCR reverse primer. Only when both TSP-L and TSP-T are hybridized to their respective targets, can they be ligated into a complete probe. The ligated probe contains a target-specific, uniquely pre-designed ratio of binding sites for red and green fluorescent probes, and serves as the template for subsequent PCR to amplify the number of fluorescent probe binding sites at the ratio encoded in each pair of TSP-L and TSP-R.

The assay starts with the mixture of TSP-Ls and TSP-Rs and nucleic acid targets. When binding to their respective target, the 3' end of TSP-L and the phosphorylated 5' end of TSP-R are brought into proximity that leads to formation of a complete linear probe via ligation. The ligated probe contains a uniquely pre-designed ratio of binding sites for red and green fluorescent probes as well as common primer binding regions on both ends. Subsequently, PCR is performed to generate amplicons with sequences complementary to the ligated probe, serving as binding sites for fluorescent probes. Upon hybridization with red and green fluorescent probes, the fluorophores-labeled amplification products are readout in both green and red channels using standard plate reader, microfluidic digital detection or single-molecule detection modalities. The measured R/G fluorescence ratios can be used to identify DNA targets in the initial input.

Probes not bound to a nucleic acid sequence and that are not ligated are not amplified exponentially in PCR. Therefore in the presence of targets of interest, only the ligated probes are amplified via PCR to generate DNA templates for fluorescent probes binding. If the unbound TSP-Ls and TSP-Rs are required to be removed, there are various approaches to purify the ligated complete probes, such as High Performance Liquid Chromatography (HPLC) and Polyacrylamide Gel Electrophoresis (PAGE).

In the examples discussed throughout the specification, numerous ligation approaches can be used and envisioned. Two non-limiting examples that allow TSPs to be ligated in the presence of their respective nucleic acid targets are as follow. In one approach, upon hybridization of TSPs to the target sequence, the phosphorylated 5' end and the 3' end of the TSP are brought into proximity. Then a DNA ligase seals the perfectly matched base-pair at the nick and generate a ligated probe for subsequent amplification[34, 35]. In another approach, there is a gap of a defined number of nucleotides between the phosphorylated 5' end and the 3' end of TSP after hybridization onto the target sequence, where there are two variants for gap filling. In the first version, a short probe oligonucleotide of the same length as the gap is hybridized between the phosphorylated 5' end and the 3' end of the TSP. Then ligation is performed using DNA ligase to generate a ligated probe for subsequent amplification[36, 37]. In the second version, dNTPs are incorporated via enzymatic polymerization proceeding from the 3' end of the TSP and terminating at the junction with the phosphorylated 5' end. Then a DNA ligase is used to seal the nick and generate a ligated probe for subsequent amplification [36, 38].

*Example 11*

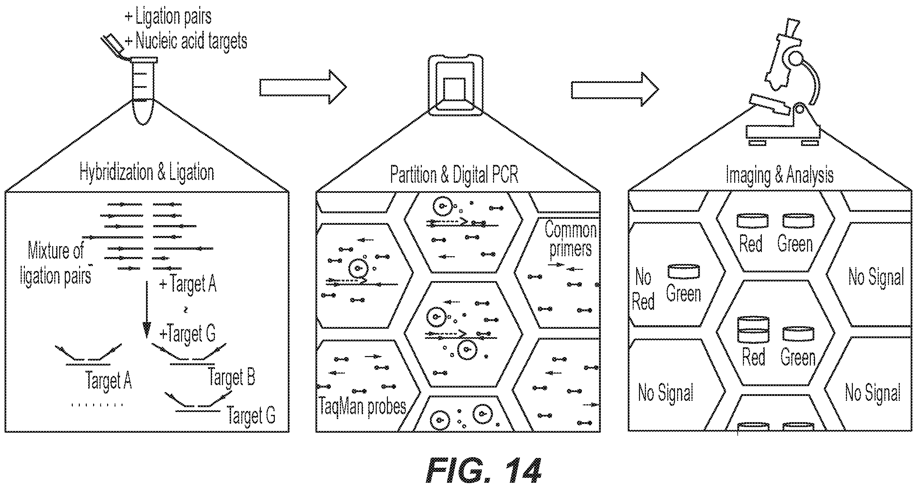

PCR is ubiquitously employed in molecular diagnostics to enable the detection of trace levels of nucleic acid targets. Most PCR-based diagnostic assays rely upon a fluorescence-based detection scheme. However, traditional fluorescence systems are limited to three, and no more than five, colors, thereby precluding the ability to perform highly multiplexed analyses. Herein, we describe a ligation-based multiplex nucleic acid amplification scheme that maximizes the multiplexing capability of standard fluorescence-based PCR assays by encoding each target-specific ligation pair with a distinct fluorescence signature. Each ligation pair is hybridized to its complementary target and covalently linked under enzymatic reaction. The ligation product then serves as a template for subsequent PCR reaction, generating specific fluorescence code for each target. The ligation products can be partitioned into different chambers of digital array chip, followed by single-molecule PCR amplification. Each chamber containing different ligation products will generate its distinct end-point signal (Figure 14).

Figure 14 is a schematic illustration of ligation-based multiplex nucleic acid detection method with fluorescence coding. The overall process starts with a ligation step with a mixture of ligation pairs in the presence of the target mixture. Subsequently, TaqMan digital PCR reaction is performed to generate distinct fluorescence signals in each chamber of digital chip. End-point fluorescence can be measured to identify and quantify each DNA target in the initial input with distinct fluorescence codes.

Ten ligation pairs targeting ten different loci of ovarian cancer methylation biomarkers were synthesized. Each ligation pair contains universal primer and TaqMan probe binding sites to achieve a distinct fluorescent signal upon amplification. As a proof of concept, ligation reactions under constant reaction solution containing a mixture of ten ligation pairs were carried out in the presence of each target, followed by subsequent TaqMan PCR. The end-point fluorescence signals were then measured on a Typhoon image scanner (GE Healthcare). Multiplex detection was achieved by TaqMan digital PCR on QuantStudioTM 3D digital PCR 20K chip (Applied Biosystems), followed by end-point fluorescence detection on an AxioZoom V16 Fluorescence Stereoscope (Zeiss).

The method was demonstrated by incorporating varying numbers of fluorescein and Alexa-555 TaqMan probe binding sites into ten ligation pairs targeting ten different ovarian cancer methylation biomarker loci. A design strategy for achieving specific fluorescence signals without non-specific amplification was developed and validated (data not shown).

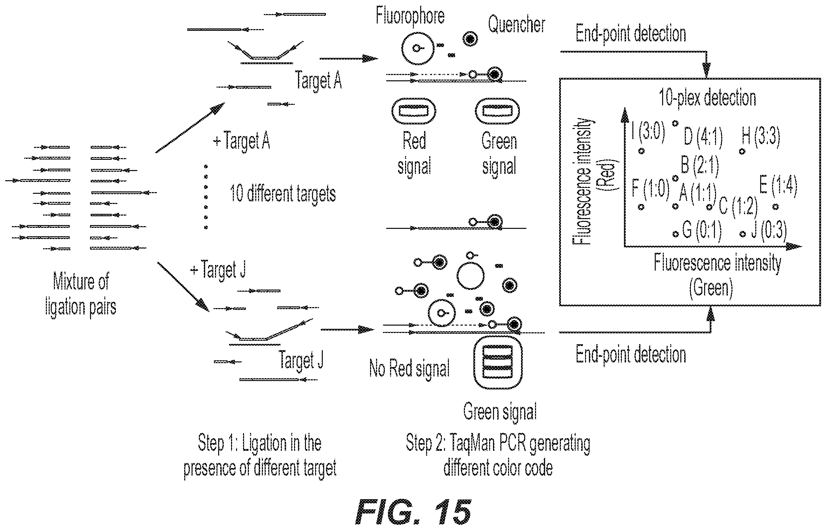

In order to demonstrate the performance of the 10-plex detection system via only two different fluorescently labeled probes, each of ten different targets were used in each ligation reaction. Each ligation product was used as a template for subsequent TaqMan PCR, and the end-point fluorescence signals from each PCR reaction were measured (Figure 15). Figure 15 shows experimental steps for demonstrating the performance of the present method. The mixture of ten ligation pairs was used in the ligation step in the presence of each of ten different targets, followed by subsequent TaqMan PCR reaction for generation distinct fluorescence signals. End-point fluorescence signals from each PCR product were measured to verify that each ligation pair generated distinct fluorescence codes.

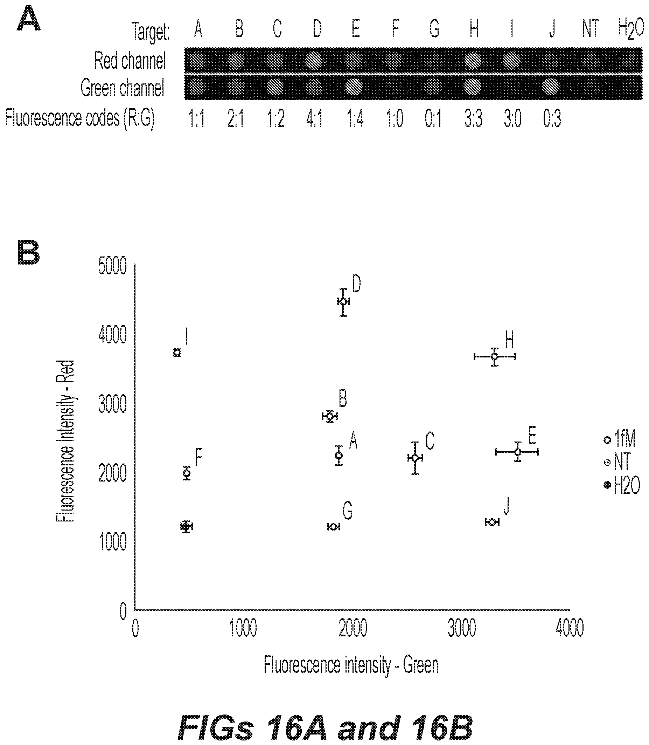

As shown in Figures 16A and 16B, each ligation product in the presence of ten different targets generated its distinct fluorescence signal that was easily differentiated from other fluorescence signals. Furthermore, the ligation-based detection system showed no detectable crosstalk between different target strands. Figure 16A shows ligation -based PCR analysis for 10-plex detection method was validated via detecting 10 ovarian cancer biomarker sequences. 1fM of each target was used in ligation step, and designed fluorescence codes were shown below the plate image. NT is a no target sample in ligation reaction further used as PCER template, and H2O is a water control for TaqMan PCR reaction step. Figure 16B shows the assay was triplicated to generate distinct fluorescence codes with error bars, indicating a robust reproducibility. The error bars represent standard deviations of red (vertical) and green (horizontal) signals.

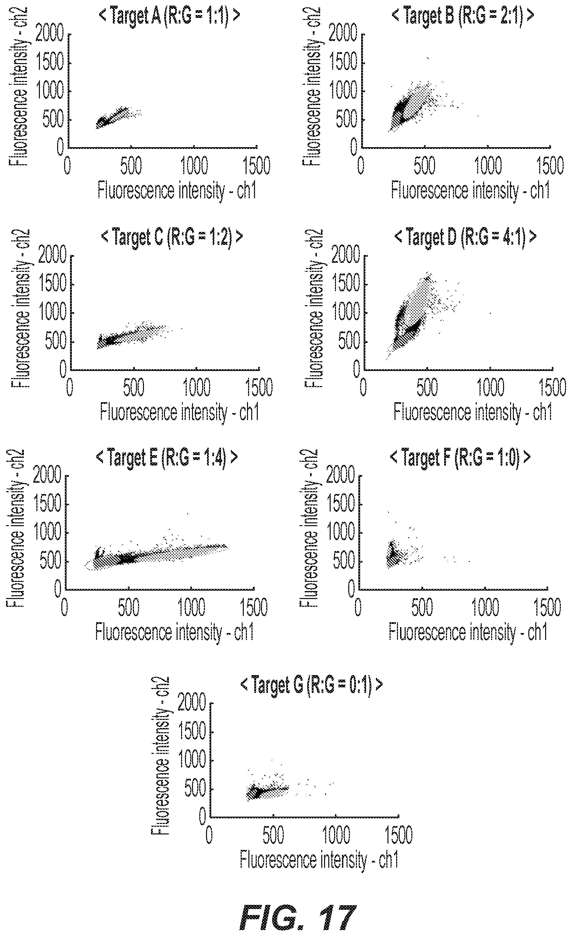

The optimized reaction condition was further applied in TaqMan digital PCR for the simultaneous detection of seven different methylation biomarkers of ovarian cancer. The target detection regions for each target were statistically defined with a 95% confidence level from bivariate distributions of fluorescence signals generated by each ligation product (Figure 17). Figure 17 shows seven distinct fluorescence patterns generated from each ligation product in TaqMan digital PCR reactions. Each data point in the scatter plot represents the fluorescence signal from each chamber in digital chip. Seven different ligation products generated from seven different targets showed distinct bivariate distribution patterns of positive signals. From each patter, target detection regions were statistically defined with 95% confidence level.

These target detection regions were then combined to simultaneously detect different concentrations of target DNA mixtures (Figure 5). 10 fM of each target DNA strand resulted in multiple copies of ligation products in each chamber of the digital chip, generating a mixture of fluorescence coding signals. However, as the target mixture was diluted to 100 aM, the ligation products were successfully digitized and further generated its distinct fluorescence signals. The numbers of data points in each target detection region from 100 aM of each target indicated the sensitivity of the present method less than 100 aM.

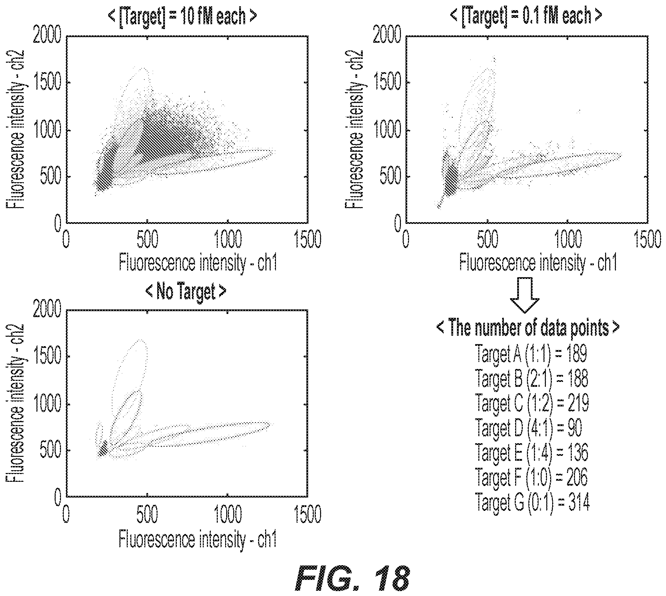

Figure 18 shows simultaneous detection of seven different targets with ligation-based TaqMan digital PCR analysis. Random fluorescence signals generated from the large amount of ligation products (10fM of each target) were due to the multiple copies of ligation products in digital PCR chambers. Well-digitized ligation products from 0.1fM of each target showed seven distinct fluorescent signals, whereas no significant numbers of data points were observed in no target sample. The number of data points from 0.1fM of each target were done below the scatter plot.

The present study successfully demonstrated a novel approach to achieve high multiplex capability of nucleic acid detection system. The result showed that the detection system could successfully discriminate ten different target DNA sequences using only two fluorescent probes. Furthermore, simultaneous detection of seven different target DNA sequences with < 100 aM sensitivity was achieved by utilizing TaqMan digital PCR for single-molecule detection of different ligation products. It is expected that the same paradigm can be readily extended to at least 3 colors to attain multiplexing of 100-plex or more.

**References**

1. Moreno, J. G.; Croce, C. M.; Fischer, R.; Monne, M.; Vihko, P.; Mulholland, S. G.; Gomella, L. G., Detection of Hematogenous Micrometastasis in Patients with Prostate-Cancer. Cancer Res 1992, 52 (21), 6110-6112.
2. Bernard, P. S.; Wittwer, C. T., Real-time PCR technology for cancer diagnostics. Clin Chem 2002, 48 (8), 1178-1185.
3. Boehm, C. D., Use of Polymerase Chain-Reaction for Diagnosis of Inherited Disorders. Clin Chem 1989, 35 (9), 1843-1848.
4. He, L. P.; Chinnery, P. F.; Durham, S. E.; Blakely, E. L.; Wardell, T. M.; Borthwick, G. M.; Taylor, R. W.; Turnbull, D. M., Detection and quantification of mitochondrial DNA deletions in individual cells by real-time PCR. Nucleic Acids Res 2002, 30 (14).
5. Monis, P. T.; Giglio, S., Nucleic acid amplification-based techniques for pathogen detection and identification. Infect Genet Evol 2006, 6 (1), 2-12.
6. Higuchi, R.; Dollinger, G.; Walsh, P. S.; Griffith, R., Simultaneous Amplification and Detection of Specific DNA-Sequences. Bio-Technol 1992, 10 (4), 413-417.
7. Wittwer, C. T.; Herrmann, M. G.; Moss, A. A.; Rasmussen, R. P., Continuous fluorescence monitoring of rapid cycle DNA amplification. Biotechniques 1997, 22 (1), 130-&.

8. Landegren, U.; Kaiser, R.; Sanders, J.; Hood, L., A Ligase-Mediated Gene Detection Technique. Science 1988, 241 (4869), 1077-1080.

9. Abravaya, K.; Carrino, J. J.; Muldoon, S.; Lee, H. H., Detection of Point Mutations with a Modified Ligase Chain-Reaction (Gap-Lcr). Nucleic Acids Res 1995, 23 (4), 675-682.

10. Compton, J., Nucleic-Acid Sequence-Based Amplification. Nature 1991, 350 (6313), 91-92.

11. Heim, A.; Grumbach, I. M.; Zeuke, S.; Top, B., Highly sensitive detection of gene expression of an intronless gene: amplification of mRNA, but not genomic DNA by nucleic acid sequence based amplification (NASBA). Nucleic Acids Res 1998, 26 (9), 2250-2251.

12. Eriksson, R.; Jobs, M.; Ekstrand, C.; Ullberg, M.; Herrmann, B.; Landegren, U.; Nilsson, M.; Blomberg, J., Multiplex and quantifiable detection of nucleic acid from pathogenic fungi using target-specific probes, generic real time PCR and specific suspension array readout. J Microbiol Meth 2009, 78 (2), 195-202.

13. Elenitoba-Johnson, K. S. J.; Bohling, S. D.; Wittwer, C. T.; King, T. C., Multiplex PCR by multicolor fluorimetry and fluorescence melting curve analysis. Nat Med 2001, 7 (2), 249-253.

14. Liao, Y. Q.; Wang, X. B.; Sha, C.; Xia, Z. M.; Huang, Q. Y.; Li, Q. G., Combination of fluorescence color and melting temperature as a two-dimensional label for homogeneous multiplex PCR detection. Nucleic Acids Res 2013, 41 (7).

15. Schouten, J. P.; McElgunn, C. J.; Waaijer, R.; Zwijnenburg, D.; Diepvens, F.; Pals, G., Relative quantification of 40 nucleic acid sequences by multiplex ligation-dependent probe amplification. Nucleic Acids Res 2002, 30 (12).

16. Chung, B.; Shin, G. W.; Na, J.; Oh, M. H.; Jung, G. Y., Multiplex quantitative foodborne pathogen detection using high resolution CE-SSCP coupled stuffer-free multiplex ligation-dependent probe amplification. Electrophoresis 2012, 33 (9-10), 1477-1481.

17. Huang, Q. Y.; Zheng, L. L.; Zhu, Y. M.; Zhang, J. F.; Wen, H. X.; Huang, J. W.; Niu, J. J.; Zhao, X. L.; Li, Q. G., Multicolor Combinatorial Probe Coding for Real-Time PCR. Plos One 2011, 6 (1).

18. Huang, Q. Y.; Hu, Q. H.; Li, Q. G., Identification of 8 foodborne pathogens by multicolor combinational probe coding technology in a single real-time PCR. Clin Chem 2007, 53 (10), 1741-1748.

19. Rajagopal, A.; Scherer, A.; Homyk, A.; Kartalov, E., Supercolor coding methods for large-scale multiplexing of biochemical assays. Anal Chem 2013, 85 (16), 7629-36.

20. Liu, D. Y.; Daubendiek, S. L.; Zillman, M. A.; Ryan, K.; Kool, E. T., Rolling circle DNA synthesis: Small circular oligonucleotides as efficient templates for DNA polymerases. J Am Chem Soc 1996, 118 (7), 1587-1594.

21. Lizardi, P. M.; Huang, X. H.; Zhu, Z. R.; Bray-Ward, P.; Thomas, D. C.; Ward, D. C., Mutation detection and single-molecule counting using isothermal rolling-circle amplification. Nat Genet 1998, 19 (3), 225-232.

22. Schweitzer, B.; Wiltshire, S.; Lambert, J.; O'Malley, S.; Kukanskis, K.; Zhu, Z. R.; Kingsmore, S. F.; Lizardi, P. M.; Ward, D. C., Immunoassays with rolling circle DNA amplification: A versatile platform for ultrasensitive antigen detection. P Natl Acad Sci USA 2000, 97 (18), 10113-10119.

23. Dean, F. B.; Nelson, J. R.; Giesler, T. L.; Lasken, R. S., Rapid amplification of plasmid and phage DNA using phi29 DNA polymerase and multiply-primed rolling circle amplification. Genome Res 2001, 11 (6), 1095-1099.

24. Nielsen, P. E.; Egholm, M., An introduction to peptide nucleic acid. Curr Issues Mol Biol 1999, 1 (1-2), 89-104.

25. Demidov, V. V.; Yavnilovich, M. V.; Belotserkovskii, B. P.; Frankkamenetskii, M. D.; Nielsen, P. E., Kinetics and Mechanism of Polyamide (Peptide) Nucleic-Acid Binding to Duplex DNA. P Natl Acad Sci USA 1995, 92 (7), 2637-2641.

26. Ali, M. M.; Li, F.; Zhang, Z. Q.; Zhang, K. X.; Kang, D. K.; Ankrum, J. A.; Le, X. C.; Zhao, W. A., Rolling circle amplification: a versatile tool for chemical biology, materials science and medicine. Chem Soc Rev 2014, 43 (10), 3324-3341.

27. Demidov, V. V., Rolling-circle amplification in DNA diagnostics: the power of simplicity. Expert Rev Mol Diagn 2002, 2 (6), 542-8.

28. Berlier, J. E.; Rothe, A.; Buller, G.; Bradford, J.; Gray, D. R.; Filanoski, B. J.; Telford, W. G.; Yue, S.; Liu, J.; Cheung, C. Y.; Chang, W.; Hirsch, J. D.; Beechem, J. M.; Haugland, R. P.; Haugland, R. P., Quantitative comparison of long-wavelength Alexa Fluor dyes to Cy dyes: fluorescence of the dyes and their bioconjugates. J Histochem Cytochem 2003, 51 (12), 1699-712.

29. Anderson, G. P.; Nerurkar, N. L., Improved fluoroimmunoassays using the dye Alexa Fluor 647 with the RAPTOR, a fiber optic biosensor. J Immunol Methods 2002, 271 (1-2), 17-24.

30. Hsieh, C. C.; Doyle, P. S., Studying confined polymers using single-molecule DNA experiments. Korea-Aust Rheol J 2008, 20 (3), 127-142.

31. Araki, S.; Nakai, T.; Hizume, K.; Takeyasu, K.; Yoshikawa, K., Hydrodynamic radius of circular DNA is larger than that of linear DNA. Chem Phys Lett 2006, 418 (1-3), 255-259.

32. Li, H. T.; Ying, L. M.; Green, J. J.; Balasubramanian, S.; Klenerman, D., Ultrasensitive coincidence fluorescence detection of single DNA molecules. Anal Chem 2003, 75 (7), 1664-1670.

33. Liu, K. J.; Wang, T. H., Cylindrical illumination confocal spectroscopy: Rectifying the limitations of confocal single molecule spectroscopy through one-dimensional beam shaping. Biophys J 2008, 95 (6), 2964-2975.

34. Larsson, C.; Koch, J.; Nygren, A.; Janssen, G.; Raap, A. K.; Landegren, U.; Nilsson, M. Nat Methods 2004, 1, 227-232.

35. Shaposhnikov, S.; Larsson, C.; Henriksson, S.; Collins, A.; Nilsson, M. Mutagenesis 2006, 21, 243-247.

36. Lizardi, P. M.; Huang, X. H.; Zhu, Z. R.; Bray-Ward, P.; Thomas, D. C.; Ward, D. C. Nat Genet 1998, 19, 225-232.

37. Mignardi, M.; Mezger, A.; Qian, X. Y.; La Fleur, L.; Botling, J.; Larsson, C.; Nilsson, M. Nucleic Acids Res 2015, 43.

38. Hardenbol, P.; Baner, J.; Jain, M.; Nilsson, M.; Namsaraev, E. A.; Karlin-Neumann, G. A.; Fakhrai-Rad, H.; Ronaghi, M.; Willis, T. D.; Landegren, U.; Davis, R. W. Nat Biotechnol 2003, 21, 673-678.

## Claims

1. A method for multiplexed detection of a nucleic acid sequence in a sample comprising:

   obtaining a plurality of oligonucleotide target-specific probes (TSPs), wherein each of the TSPs is configured to bind to a distinct target nucleic acid sequence, and wherein each of the TSPs comprises:

   at least one target-binding region configured to bind to at least a portion of the distinct target nucleic acid sequence;
   at least one common primer-binding region; and
   one or more copies of a first fluorescent probe (FP) binding region and one or more copies of a second FP binding region, wherein a predetermined ratio of the one or more copies of the first FP binding region to the one or more copies of the second FP binding region is indicative of the distinct target nucleic acid sequence the TSP is configured to bind to;

   contacting the plurality of TSPs with the nucleic acid sequence in the sample such that at least one of the TSPs binds to at least a portion of the nucleic acid sequence and such that a TSP-nucleic acid sequence complex is formed;
   ligating the at least one of the TSPs bound to at least a portion of the nucleic acid sequence such that a ligated TSP is formed;
   generating target-specific oligonucleotide sequences (TSSs) by a nucleic acid amplification assay, and wherein the ligated TSP is a template for the nucleic acid amplification assay;
   contacting the TSSs with a plurality of differently labeled fluorescent probes (FPs) such that a TSS-FP complex comprising the TSS and at least one of the first FP and the second FP is generated;
   measuring a fluorescence ratio; and
   identifying the nucleic acid sequence based on the florescence ratio,
   wherein the FPs comprise linear oligonucleotide probes labeled with fluorophores and
   molecule beacons (MBs) that are hairpin shaped oligonucleotide probes labeled with fluorophores and quenchers, and wherein MBs fluorescence is quenched in a native state and restored upon hybridization to the TSSs.

2. The method of claim 1, wherein each of the TSPs further comprises:

   a first target-binding region at a first end of the TSP, wherein the first target-binding region binds to at least a portion of the distinct target nucleic acid sequence; and
   a second target-binding region at a second end of the TSP, wherein the second target-binding region binds to at least a portion of the distinct target nucleic acid sequence,
   wherein the ligating the at least one of the TSPs bound to at least a portion of the nucleic acid sequence results in a circularized TSP, and
   wherein the circularized TSP is the template for the nucleic acid amplification assay.

3. The method of claim 1, wherein the plurality of oligonucleotide target-specific probes (TSPs), comprises a plurality of TSP pairs configured to bind to a distinct target nucleic acid sequence, and wherein each pair of the plurality of TSP pairs comprises:

   a first TSP comprising:

   a target-specific binding region configured to bind to at least a first portion of the distinct target nucleic acid sequence, wherein the first target-specific binding region is located at a 3' end of the first TSP;
   at least one common primer-binding region; and

one or more copies of a first fluorescent probe (FP) binding region; and

a second TSP comprising:

a target-specific binding region configured to bind to at least a second portion of the distinct target nucleic acid sequence, wherein the target-specific binding region is located at a 5' end of the second TSP;
at least one common primer-binding region; and
one or more copies of a second fluorescent probe (FP) binding region,

wherein the first portion of the distinct target nucleic acid sequence and the second portion of the distinct target nucleic acid sequence are adjacent, and
wherein a predetermined ratio of the one or more copies of the first FP binding region to the one or more copies of the second FP binding region is indicative of the distinct target nucleic acid sequence the pair is configured to bind to.

4. The method of claim 1,

(i) wherein the FPs comprise oligonucleotides sequences comprising one or more of nucleic acids, nucleic acid analogues including peptide nucleic acids (PNAs), and locked nucleic acids (LNAs),
(ii) wherein the nucleic acid amplification assay is selected from the list consisting of rolling circle amplification (RCA), hyperbranched rolling circle amplification (HRCA), and polymerase chain reaction (PCR), or
(iii) wherein the sample comprises one unidentified nucleic acid target sequence out of multiple candidates.

5. The method of claim 1, further comprising (i) removing TSPs that are not bound to at least a portion of the nucleic acid sequence following the ligating of the at least one of the TSPs bound to at least a portion of the nucleic acid sequence,

(ii) removing FPs not hybridized to the TSSs comprising the use a purification spin column, or
(iii) the use of a single molecule detection (SMD) system, and measuring a photon count of each fluorescence color of a single TSS-FP complex comprising the use of cylindrical illustration confocal spectroscopy (CICS).

6. The method of claim 5, wherein (i) the SMD system comprises a microfluidic chip comprising:

a gas permeable silicone material comprising polydimethylsiloxane (PDMS);
a transport chamber comprising at least 2 parallel flow channels, each flow channel having
a dimension of about 5 $\mu$m × 0.5 $\mu$m (width × height); and
a filter array at an inlet to reduce flow channel clogging,
wherein the microfluidic chip is used for measuring fluorescence of the TSS-TPs complex, preferably
wherein the sample is driven through the microfluidic chip using a nitrogen pressure source, and wherein the nitrogen pressure source is regulated by a series of precision gas regulators, or

(ii) the photon count of each fluorescence color of the TSS- FP complex is measured on CICS, and wherein the nucleic acid sequence is identified by a ratio of measured fluorescence photon counts.

7. The method of claim 1, further comprising:

loading a plurality of ligated TSPs, a nucleic acid amplification reaction mixture, and FPs onto an array of discrete reaction receptacles, such that each reaction receptacle contains up to one ligated TSP;
generating TSSs in each reaction receptacle by nucleic acid amplification using the ligated TSP as a template;
binding the TSSs with a plurality of differently labeled FPs in each reaction receptacle;
measuring a fluorescence ratio in each of the reaction receptacles; and
identifying the nucleic acid sequence based on the florescence ratio, preferably

(i) wherein the sample contains a plurality of nucleic acid sequences or
(ii) wherein the array of discrete reaction receptacles are located on a microfluidic chip, or wherein the array of discrete reaction receptacles are a plurality of droplets.

8. The method of claim 7, wherein (i) the chip further comprises:

a microfluidic flow chamber comprising one or more flow channels; and
a plurality of picowells with dimensions in the range of 100pL to 10nL,
wherein the one or more flow channels are in contact with the plurality of picowells, preferably
further comprising:

loading the plurality of ligated TSPs, nucleic acid amplification reaction mixture and a plurality of differently labeled MBs onto the one or more flow channels of the microfluidic chip such that each of the plurality of picowells contains up to one ligated TSP;
injecting fluid comprising oil and polydimethylsiloxane (PDMS) into the one or more flow channels but not into the plurality of picowells, such that a digital reaction well is formed;
generating TSSs in each of the plurality of picowells containing up to one ligated TSP by nucleic acid amplification using the ligated TSP as a template;
binding the TSSs with the differently labeled MBs;
measuring a fluorescence ratio; and
identifying the nucleic acid sequence based on the florescence ratio,

(i) each of the plurality of droplets have a volume of between about 5pL to 1nL,
(ii) wherein the plurality of droplets are generated on a microfluidic chip comprising:

a droplet generation module comprising flow channels, microvalves and a flow focusing junction; and
a droplet measurement module comprising flow channels, microvalves and a flow constriction channel,

(iii) wherein a custom confocal microscope system is used for measuring a fluorescence intensity in the plurality of droplets, the custom confocal microscope comprising:

a 488nm laser and a 545 nm laser;
a plurality of dichroic mirrors to combine two laser beams;
a 40× microscope objective to focus a laser beam and to collect an emitted fluorescence signal from the plurality of droplets;
a plurality of dichroic mirrors and band-pass filters to spectrally separate a desired emission fluorescence signal; and
at least 2 two avalanche photodiodes (APDs) to collect fluorescence data, or

(iv) further comprising:

loading a mixture comprising the plurality of ligated TSPs, nucleic acid amplification reaction mixture, and FPs into a droplet generation module on the chip;
loading fluid comprising an oil and a surfactant into the droplet generation module simultaneously such that the mixture is sheared into a plurality of droplets, and wherein each droplet contains up to one ligated TSP;
collecting the plurality of droplets;
incubating the collected plurality of droplets on a thermal cycler and generating TSSs by nucleic acid amplification using the ligated TSP as a template;
binding the TSSs with the differently labeled FPs;
loading the plurality of droplets into a droplet measurement module on the chip;
measuring a fluorescence ratio using a custom confocal microscope system; and
identifying the nucleic acid sequence based on the measured fluorescence ratio.

9. The method of claim 1, wherein each of the TSPs further comprises one or more copies of a third fluorescent probe (FP) binding region, wherein a predetermined ratio of the one or more copies of the first FP binding region to the one or more copies of the second FP binding region and to the one or more copies of the third FP binding region is indicative of the distinct target nucleic acid sequence the TSP is configured to bind to.

10. A kit for multiplexed detection of a nucleic acid sequence in a sample comprising:

a plurality of oligonucleotide target-specific probes (TSPs), wherein each of the TSPs is configured to bind to a distinct target nucleic acid sequence, and wherein each of the TSPs comprises:

at least one target-binding region configured to bind to at least a portion of the distinct target nucleic acid sequence;
at least one common primer-binding region; and
one or more copies of a first fluorescent probe (FP) binding region and one or more copies of a second FP binding region, wherein a predetermined ratio of the one or more copies of the first FP binding region to the one or more copies of the second FP binding region is indicative of the distinct target nucleic acid sequence the TSP is configured to bind to; and

instructions for determining the identity of the nucleic acid sequence based on a measured fluorescence ratio, wherein the FPs comprise linear oligonucleotide probes labeled with fluorophores and molecule beacons (MBs) that are hairpin shaped oligonucleotide probes labeled with fluorophores and quenchers, and wherein MBs fluorescence is quenched in a native state and restored upon hybridization to the TSSs.

11. The kit of claim 10, wherein

(i) each of the TSPs further comprises:

a first target-binding region at a first end of the TSP, wherein the first target-binding region binds to at least a portion of the distinct target nucleic acid sequence; and
a second target-binding region at a second end of the TSP, wherein the second target-binding region binds to at least a portion of the distinct target nucleic acid sequence or

(ii) the plurality of oligonucleotide target-specific probes (TSPs), comprises a plurality of TSP pairs configured to bind to a distinct target nucleic acid sequence, and wherein each pair of the plurality of TSP pairs comprises:

a first TSP comprising:

a target-specific binding region configured to bind to at least a first portion of the distinct target nucleic acid sequence, wherein the first target-specific binding region is located at a 3' end of the first TSP;
at least one common primer-binding region; and
one or more copies of a first fluorescent probe (FP) binding region; and

a second TSP comprising:

a target-specific binding region configured to bind to at least a second portion of the distinct target nucleic acid sequence, wherein the target-specific binding region is located at a 5' end of the second TSP;
at least one common primer-binding region; and
one or more copies of a second fluorescent probe (FP) binding region,

wherein the first portion of the distinct target nucleic acid sequence and the second portion of the distinct target nucleic acid sequence are adjacent, and
wherein a predetermined ratio of the one or more copies of the first FP binding region to the one or more copies of the second FP binding region is indicative of the distinct target nucleic acid sequence the pair is configured to bind to.

12. The kit of claim 10, further comprising (i) a primer which binds to the common primer-binding region, or (ii) reagents for conducting a nucleic acid amplification assay, preferably wherein the reagents for conducting a nucleic acid amplification assay are reagents suitable for conducting a rolling circle amplification assay, a hyperbranched rolling circle amplification assay, or a polymerase chain reaction assay.

13. The kit of claim 10, further comprising the first FP and the second FP.

14. The kit of claim 13, wherein the first FP and the second FP comprise oligonucleotides sequences comprising one or more of nucleic acids, nucleic acid analogues including peptide nucleic acids (PNAs), and locked nucleic acids (LNAs).

**15.** The kit of claim 10, further comprising an enzyme for removing or inactivating oligonucleotide target-specific probes that do not bind to the nucleic acid.

**Patentansprüche**

**1.** Verfahren zum gebündelten Nachweis einer Nucleinsäuresequenz in einer Probe, umfassend:

das Gewinnen einer Vielzahl von Oligonucleotid-Target-spezifischen Sonden (TSPs), wobei jede der TSPs so konfiguriert ist, dass sie an eine unterschiedliche Target-Nucleinsäuresequenz binden kann, und wobei jede der TSPs Folgendes umfasst:

wenigstens einen Target-bindenden Bereich, der so konfiguriert ist, dass er an wenigstens einen Teil der unterschiedlichen Target-Nucleinsäuresequenz binden kann;
wenigstens einen gemeinsamen Primer-bindenden Bereich; und
eine oder mehrere Kopien eines ersten Fluoreszenzsonden(FP)-bindenden Bereichs und eine oder mehrere Kopien eines zweiten FP-bindenden Bereichs, wobei ein vorbestimmtes Verhältnis der einen oder der mehreren Kopien des ersten FP-bindenden Bereichs zu der einen oder den mehreren Kopien des zweiten FP-bindenden Bereichs die unterschiedliche Target-Nucleinsäuresequenz anzeigt, an die zu binden die TSP konfiguriert ist;
das In-Kontakt-Bringen der Vielzahl von TSPs mit der Nucleinsäuresequenz in der Probe, so dass wenigstens eine der TSPs an wenigstens einen Teil der Nucleinsäuresequenz bindet und ein TSP-Nucleinsäuresequenz-Komplex entsteht;
das Ligieren der wenigstens einen der TSPs, die an wenigstens einen Teil der Nucleinsäuresequenz gebunden ist, so dass eine ligierte TSP entsteht;
das Erzeugen von Target-spezifischen Oligonucleotidsequenzen (TSSs) durch einen Nucleinsäure-Amplifikationsassay, wobei die ligierte TSP eine Matrize für den Nucleinsäure-Amplifikationsassay ist;
das In-Kontakt-Bringen der TSSs mit einer Vielzahl von unterschiedlich markierten Fluoreszenzsonden (FPs), so dass ein TSS-FP-Komplex, der die TSS und die erste FP und/oder die zweite FP umfasst, erzeugt wird;
das Messen eines Fluoreszenzverhältnisses; und
das Identifizieren der Nucleinsäuresequenz anhand des Fluoreszenzverhältnisses,
wobei die FPs lineare Oligonucleotidsonden, die mit Fluorophoren markiert sind, und Molekül-Beacons (MBs), bei denen es sich um haarnadelförmige Oligonucleotidsonden handelt, die mit Fluorophoren und Quenchern markiert sind, umfassen und wobei die Fluoreszenz der MBs in einem nativen Zustand ausgelöscht ist und bei Hybridisierung an die TSSs wiederhergestellt wird.

**2.** Verfahren gemäß Anspruch 1, wobei jede der TSPs weiterhin Folgendes umfasst:

einen ersten Target-bindenden Bereich an einem ersten Ende der TSP, wobei der erste Target-bindende Bereich an wenigstens einen Teil der unterschiedlichen Target-Nucleinsäuresequenz bindet; und
einen zweiten Target-bindenden Bereich an einem zweiten Ende der TSP, wobei der zweite Target-bindende Bereich an wenigstens einen Teil der unterschiedlichen Target-Nucleinsäuresequenz bindet,
wobei das Ligieren der wenigstens einen der TSPs, die an wenigstens einen Teil der Nucleinsäuresequenz gebunden ist, zu einer zirkularisierten TSP führt und
wobei die zirkularisierte TSP die Matrize für den Nucleinsäure-Amplifikationsassay ist.

**3.** Verfahren gemäß Anspruch 1, wobei die Vielzahl der Oligonucleotid-Target-spezifischen Sonden (TSPs) eine Vielzahl von TSP-Paaren umfasst, die so konfiguriert sind, dass sie an eine unterschiedliche Target-Nucleinsäuresequenz binden können, und wobei jedes Paar der Vielzahl von TSP-Paaren Folgendes umfasst:

eine erste TSP, umfassend:

einen Target-spezifischen Bindungsbereich, der so konfiguriert ist, dass er an wenigstens einen ersten Teil der unterschiedlichen Target-Nucleinsäuresequenz binden kann, wobei sich der erste Target-spezifische Bindungsbereich an einem 3'-Ende der ersten TSP befindet;
wenigstens einen gemeinsamen Primer-bindenden Bereich; und
eine oder mehrere Kopien eines ersten Fluoreszenzsonden(FP)-bindenden Bereichs; und

eine zweite TSP, umfassend:

einen Target-spezifischen Bindungsbereich, der so konfiguriert ist, dass er an wenigstens einen zweiten Teil der unterschiedlichen Target-Nucleinsäuresequenz binden kann, wobei sich der Target-spezifische Bindungsbereich an einem 5'-Ende der zweiten TSP befindet;
wenigstens einen gemeinsamen Primer-bindenden Bereich; und
eine oder mehrere Kopien eines zweiten Fluoreszenzsonden(FP)-bindenden Bereichs,

wobei der erste Teil der unterschiedlichen Target-Nucleinsäuresequenz und der zweite Teil der unterschiedlichen Target-Nucleinsäuresequenz benachbart sind und
wobei ein vorbestimmtes Verhältnis der einen oder der mehreren Kopien des ersten FP-bindenden Bereichs zu der einen oder den mehreren Kopien des zweiten FP-bindenden Bereichs die unterschiedliche Target-Nucleinsäuresequenz anzeigt, an die zu binden das Paar konfiguriert ist.

4. Verfahren gemäß Anspruch 1,

(i) wobei die FPs Oligonucleotidsequenzen umfassen, die eine oder mehrere aus Nucleinsäuren, Nucleinsäureanaloga einschließlich Peptidnucleinsäuren (PNAs) und Locked-Nucleinsäuren (LNAs) umfassen,
(ii) wobei der Nucleinsäure-Amplifikationsassay aus der Liste ausgewählt ist, die aus Rolling-Circle-Amplifikation (RCA), Hyperbranched-Rolling-Circle-Amplifikation (HRCA) und Polymerase-Kettenreaktion (PCR) besteht, oder
(iii) wobei die Probe eine einzige unidentifizierte Nucleinsäure-Target-Sequenz aus mehreren Kandidaten umfasst.

5. Verfahren gemäß Anspruch 1, weiterhin umfassend

(i) das Entfernen von TSPs, die nicht an wenigstens einen Teil der Nucleinsäuresequenz gebunden sind, nach dem Ligieren der wenigstens einen der TSPs, die an wenigstens einen Teil der Nucleinsäuresequenz gebunden ist,
(ii) das Entfernen von FPs, die nicht an die TSSs hybridisiert sind, unter Verwendung einer Reinigungs-Spin-Säule oder
(iii) die Verwendung eines Einzelmolekül-Nachweissystems (SMD) und das Messen einer Photonenzahl für jede Fluoreszenzfarbe eines einzelnen TSS-FP-Komplexes unter Verwendung von konfokaler Spektroskopie mit zylindrischer Beleuchtung (CICS).

6. Verfahren gemäß Anspruch 5, wobei

(i) das SMD-System einen Mikrofluidikchip umfasst mit:

einem gasdurchlässigen Silikonmaterial, das Polydimethylsiloxan (PDMS) umfasst;
einer Transportkammer, die wenigstens 2 parallele Durchflusskanäle umfasst, wobei jeder Durchflusskanal eine Abmessung von etwa 5 $\mu$m $\times$ 0,5 $\mu$m (Breite $\times$ Höhe) aufweist; und
einer Filteranordnung an einem Einlass zum Reduzieren der Verstopfung des Durchflusskanals,
wobei der Mikrofluidikchip verwendet wird, um die Fluoreszenz des TSS-TPs-Komplexes zu messen,
wobei vorzugsweise die Probe mit Hilfe einer Stickstoffdruckquelle durch den Mikrofluidikchip getrieben wird und wobei die Stickstoffdruckquelle durch eine Reihe von Präzisionsgasreglern reguliert wird, oder

(ii) die Photonenzahl jeder Fluoreszenzfarbe des TSS-FP-Komplexes auf CICS gemessen wird und wobei die Nucleinsäuresequenz durch ein Verhältnis von gemessenen Fluoreszenzphotonenzahlen identifiziert wird.

7. Verfahren gemäß Anspruch 1, weiterhin umfassend:

das Beladen einer Anordnung von diskreten Reaktionsbehältern mit einer Vielzahl von ligierten TSPs, einem Nucleinsäure-Amplifikationsreaktionsgemisch und FPs in einer solchen Weise, dass jeder Reaktionsbehälter bis zu genau einer ligierten TSP enthält;
das Erzeugen von TSSs in jedem Reaktionsbehälter durch Nucleinsäureamplifikation unter Verwendung der ligierten TSP als Matrize;
das Binden der TSSs an eine Vielzahl von unterschiedlich markierten FPs in jedem Reaktionsbehälter;
das Messen eines Fluoreszenzverhältnisses in jedem der Reaktionsbehälter; und
das Identifizieren der Nucleinsäuresequenz anhand des Fluoreszenzverhältnisses, wobei vorzugsweise

(i) die Probe eine Vielzahl von Nucleinsäuresequenzen enthält oder

(ii) sich die Anordnung von diskreten Reaktionsbehältern auf einem Mikrofluidikchip befindet oder wobei es sich bei der Anordnung von diskreten Reaktionsbehältern um eine Vielzahl von Tröpfchen handelt.

8. Verfahren gemäß Anspruch 7, wobei (i) der Chip weiterhin umfasst:

eine Mikrofluidik-Durchflusskammer, die einen oder mehrere Durchflusskanäle umfasst; und

eine Vielzahl von Picowells mit Abmessungen im Bereich von 100 pl bis 10 nl,

wobei sich der eine oder die mehreren Durchflusskanäle in Kontakt mit der Vielzahl von Picowells befinden, vorzugsweise

weiterhin umfassend:

das Beladen des einen oder der mehreren Durchflusskanäle des Mikrofluidikchips mit der Vielzahl von ligierten TSPs, dem Nucleinsäure-Amplifikationsreaktionsgemisch und einer Vielzahl von unterschiedlich markierten MBs in einer solchen Weise, dass jeder der Vielzahl von Picowells bis zu genau einer ligierten TSP enthält;

das Injizieren einer Flüssigkeit, die Öl und Polydimethylsiloxan (PDMS) umfasst, in den einen oder die mehreren Durchflusskanäle, aber nicht in die Vielzahl von Picowells, so dass ein digitaler Reaktionswell gebildet wird;

das Erzeugen von TSSs in jedem der Vielzahl von Picowells, die bis zu genau einer TSP enthalten, durch Nucleinsäureamplifikation unter Verwendung der ligierten TSP als Matrize;

das Binden der TSSs an die unterschiedlich markierten MBs;

das Messen eines Fluoreszenzverhältnisses; und

das Identifizieren der Nucleinsäuresequenz anhand des Fluoreszenzverhältnisses,

(i) jedes der Vielzahl von Tröpfchen ein Volumen von etwa 5 pl bis 1 nl aufweist,

(ii) wobei die Vielzahl von Tröpfchen auf einem Mikrofluidikchip erzeugt werden, umfassend:

ein Tröpfchenerzeugungsmodul, das Durchflusskanäle, Mikroventile und eine strömungsfokussierende Verbindungsstelle umfasst; und

ein Tröpfchenmessungsmodul, das Durchflusskanäle, Mikroventile und einen strömungsverengenden Kanal umfasst,

(iii) wobei ein maßgeschneidertes konfokales Mikroskopsystem verwendet wird, um eine Fluoreszenzintensität in der Vielzahl von Tröpfchen zu messen, wobei das maßgeschneiderte konfokale Mikroskop Folgendes umfasst:

einen 488-nm-Laser und einen 545-nm-Laser;

eine Vielzahl von dichroitischen Spiegeln zum Kombinieren von zwei Laserstrahlen;

ein 40×-Mikroskopobjektiv zum Fokussieren eines Laserstrahls und zum Auffangen eines emittierten Fluoreszenzsignals aus der Vielzahl der Tröpfchen;

eine Vielzahl von dichroitischen Spiegeln und Bandfiltern zum spektralen Zerlegen eines gewünschten Emissionsfluoreszenzsignals; und

wenigstens zwei Lawinenfotodioden (APDs) zum Sammeln von Fluoreszenzdaten oder

(iv) weiterhin umfassend:

das Beladen eines Tröpfchenerzeugungsmoduls auf dem Chip mit einem Gemisch, das die der Vielzahl von ligierten TSPs, das Nucleinsäure-Amplifikationsreaktionsgemisch und FPs umfasst;

das gleichzeitige Beladen des Tröpfchenerzeugungsmoduls mit einer Flüssigkeit, die ein Öl und ein Tensid umfasst, so dass das Gemisch zu einer Vielzahl von Tröpfchen auseinandergeschert wird, wobei jedes Tröpfchen bis zu genau einer ligierten TSP enthält;

das Auffangen der Vielzahl von Tröpfchen;

das Inkubieren der aufgefangenen Vielzahl von Tröpfchen auf einem Thermocycler und das Erzeugen von TSSs durch Nucleinsäureamplifikation unter Verwendung der ligierten TSP als Matrize;

das Binden der TSSs an die unterschiedlich markierten FPs;

das Beladen eines Tröpfchenmessungsmoduls auf dem Chip mit der Vielzahl von Tröpfchen;

das Messen eines Fluoreszenzverhältnisses unter Verwendung eines konfokalen Mikroskopsystems; und

das Identifizieren der Nucleinsäuresequenz anhand des gemessenen Fluoreszenzverhältnisses.

9. Verfahren gemäß Anspruch 1, wobei jede der TSPs weiterhin eine oder mehrere Kopien eines dritten Fluoreszenzsonden(FP)-bindenden Bereichs umfasst, wobei ein vorbestimmtes Verhältnis der einen oder der mehreren Kopien des ersten FP-bindenden Bereichs zu der einen oder den mehreren Kopien des zweiten FP-bindenden Bereichs und zu der einen oder den mehreren Kopien des dritten FP-bindenden Bereichs die unterschiedliche Target-Nucleinsäuresequenz anzeigt, an die zu binden die TSP konfiguriert ist.

10. Kit zum gebündelten Nachweis einer Nucleinsäuresequenz in einer Probe, umfassend:

eine Vielzahl von Oligonucleotid-Target-spezifischen Sonden (TSPs), wobei jede der TSPs so konfiguriert ist, dass sie an eine unterschiedliche Target-Nucleinsäuresequenz binden kann, und wobei jede der TSPs Folgendes umfasst:

wenigstens einen Target-bindenden Bereich, der so konfiguriert ist, dass er an wenigstens einen Teil der unterschiedlichen Target-Nucleinsäuresequenz binden kann;
wenigstens einen gemeinsamen Primer-bindenden Bereich; und
eine oder mehrere Kopien eines ersten Fluoreszenzsonden(FP)-bindenden Bereichs und eine oder mehrere Kopien eines zweiten FP-bindenden Bereichs, wobei ein vorbestimmtes Verhältnis der einen oder der mehreren Kopien des ersten FP-bindenden Bereichs zu der einen oder den mehreren Kopien des zweiten FP-bindenden Bereichs die unterschiedliche Target-Nucleinsäuresequenz anzeigt, an die zu binden die TSP konfiguriert ist; und

Anweisungen zum Bestimmen der Identität der Nucleinsäuresequenz anhand eines gemessenen Fluoreszenzverhältnisses,
wobei die FPs lineare Oligonucleotidsonden, die mit Fluorophoren markiert sind, und Molekül-Beacons (MBs), bei denen es sich um haarnadelförmige Oligonucleotidsonden handelt, die mit Fluorophoren und Quenchern markiert sind, umfassen und wobei die Fluoreszenz der MBs in einem nativen Zustand abgelöscht ist und bei Hybridisierung an die TSSs wiederhergestellt wird.

11. Kit gemäß Anspruch 10, wobei

(i) jede der TSPs weiterhin Folgendes umfasst:

einen ersten Target-bindenden Bereich an einem ersten Ende der TSP, wobei der erste Target-bindende Bereich an wenigstens einen Teil der unterschiedlichen Target-Nucleinsäuresequenz bindet; und
einen zweiten Target-bindenden Bereich an einem zweiten Ende der TSP, wobei der zweite Target-bindende Bereich an wenigstens einen Teil der unterschiedlichen Target-Nucleinsäuresequenz bindet, oder

(ii) die Vielzahl der Oligonucleotid-Target-spezifischen Sonden (TSPs) eine Vielzahl von TSP-Paaren umfasst, die so konfiguriert sind, dass sie an eine unterschiedliche Target-Nucleinsäuresequenz binden können, und wobei jedes Paar der Vielzahl von TSP-Paaren Folgendes umfasst:

eine erste TSP, umfassend:

einen Target-spezifischen Bindungsbereich, der so konfiguriert ist, dass er an wenigstens einen ersten Teil der unterschiedlichen Target-Nucleinsäuresequenz binden kann, wobei sich der erste Target-spezifische Bindungsbereich an einem 3'-Ende der ersten TSP befindet;
wenigstens einen gemeinsamen Primer-bindenden Bereich; und
eine oder mehrere Kopien eines ersten Fluoreszenzsonden(FP)-bindenden Bereichs; und

eine zweite TSP, umfassend:

einen Target-spezifischen Bindungsbereich, der so konfiguriert ist, dass er an wenigstens einen zweiten Teil der unterschiedlichen Target-Nucleinsäuresequenz binden kann, wobei sich der Target-spezifische Bindungsbereich an einem 5'-Ende der zweiten TSP befindet;

wenigstens einen gemeinsamen Primer-bindenden Bereich; und
eine oder mehrere Kopien eines zweiten Fluoreszenzsonden(FP)-bindenden Bereichs,

wobei der erste Teil der unterschiedlichen Target-Nucleinsäuresequenz und der zweite Teil der unterschiedlichen Target-Nucleinsäuresequenz benachbart sind und
wobei ein vorbestimmtes Verhältnis der einen oder der mehreren Kopien des ersten FP-bindenden Bereichs zu der einen oder den mehreren Kopien des zweiten FP-bindenden Bereichs die unterschiedliche Target-Nucleinsäuresequenz anzeigt, an die zu binden das Paar konfiguriert ist.

**12.** Kit gemäß Anspruch 10, weiterhin umfassend (i) einen Primer, der an den gemeinsamen primerbindenden Bereich bindet, oder
(ii) Reagentien zum Durchführen eines Nucleinsäure-Amplifikationsassays, wobei vorzugsweise die Reagentien zum Durchführen eines Nucleinsäure-Amplifikationsassays Reagentien sind, die zum Durchführen eines Rolling-Circle-Amplifikationsassays, eines Hyperbranched-Rolling-Circle-Amplifikationsassays oder eines Polymerase-Kettenreaktionsassays geeignet sind.

**13.** Kit gemäß Anspruch 10, weiterhin umfassend die erste FP und die zweite FP.

**14.** Kit gemäß Anspruch 13,
wobei die erste FP und die zweite FP Oligonucleotidsequenzen umfassen, die eine oder mehrere aus Nucleinsäuren, Nucleinsäureanaloga einschließlich Peptidnucleinsäuren (PNAs) und Locked-Nucleinsäuren (LNAs) umfassen.

**15.** Kit gemäß Anspruch 10, weiterhin umfassend ein Enzym zum Entfernen oder Inaktivieren Oligonucleotid-Target-spezifischer Sonden, die nicht an die Nucleinsäure binden.

**Revendications**

**1.** Procédé de détection multiplexée d'une séquence d'acide nucléique dans un échantillon comprenant :
l'obtention d'une pluralité de sondes oligonucléotidiques spécifiques à une cible (TSP), dans lequel chacune des TSP est conçue pour se lier à une séquence d'acide nucléique cible distincte, et dans lequel chacune des TSP comprend :

au moins une région de liaison à une cible conçue pour se lier à au moins une partie de la séquence d'acide nucléique cible distincte ;
au moins une région commune de liaison à une amorce ; et
une ou plusieurs copies d'une première région de liaison à une sonde fluorescente (FP) et une ou plusieurs copies d'une deuxième région de liaison FP, dans lequel un rapport prédéterminé de la ou des copies de la première région de liaison FP à la ou aux copies de la deuxième région de liaison FP indique la séquence d'acide nucléique cible distincte à laquelle la TSP est conçue pour se lier ;
la mise en contact de la pluralité de TSP avec la séquence d'acide nucléique dans l'échantillon de telle sorte qu'au moins une des TSP se lie à au moins une partie de la séquence d'acide nucléique et qu'un complexe TSP-séquence d'acide nucléique est formé ;
la ligature de l'au moins une des TSP liée à au moins une partie de la séquence d'acide nucléique de telle sorte qu'une TSP ligaturée est formée ;
la génération de séquences oligonucléotidiques spécifiques à une cible (TSS) par un essai d'amplification d'acide nucléique, et dans lequel la TSP ligaturée est une matrice pour l'essai d'amplification d'acide nucléique ;
la mise en contact des TSS avec une pluralité de sondes fluorescentes (FP) marquées différemment de telle sorte qu'un complexe TSS-FP comprenant la TSS et au moins l'une de la première FP et de la seconde FP est généré ;
la mesure d'un rapport de fluorescence ; et
l'identification de la séquence d'acide nucléique sur la base du rapport de fluorescence,
dans lequel les FP comprennent des sondes oligonucléotidiques linéaires marquées avec des fluorophores et des balises moléculaires (MB) qui sont des sondes oligonucléotidiques en forme d'épingle à cheveux marquées avec des fluorophores et des inhibiteurs, et dans lequel la fluorescence des MB est inhibée à un état natif et rétablie lors de l'hybridation aux TSS.

**2.** Procédé selon la revendication 1, dans lequel chacune des TSP comprend en outre :

une première région de liaison à une cible au niveau d'une première extrémité de la TSP, dans lequel la première région de liaison à une cible se lie à au moins une partie de la séquence d'acide nucléique cible distincte ; et une seconde région de liaison à une cible au niveau d'une seconde extrémité de la TSP, dans lequel la seconde région de liaison à une cible se lie à au moins une partie de la séquence d'acide nucléique cible distincte, dans lequel la ligature de l'au moins une des TSP liée à au moins une partie de la séquence d'acide nucléique produit une TSP circularisée, et

dans lequel la TSP circularisée est la matrice pour l'essai d'amplification d'acide nucléique.

3. Procédé selon la revendication 1, dans lequel la pluralité de sondes oligonucléotidiques spécifiques à une cible (TSP), comprend une pluralité de paires de TSP conçues pour se lier à une séquence d'acide nucléique cible distincte, et dans lequel chaque paire de la pluralité de paires de TSP comprend :

une première TSP comprenant :

une région de liaison spécifique à une cible conçue pour se lier à au moins une première partie de la séquence d'acide nucléique cible distincte, dans lequel la première région de liaison spécifique à une cible est située à une extrémité 3' de la première TSP ;
au moins une région commune de liaison à une amorce ; et
une ou plusieurs copies d'une première région de liaison à une sonde fluorescente (FP) ; et
une seconde TSP comprenant :

une région de liaison spécifique à une cible conçue pour se lier à au moins une seconde partie de la séquence d'acide nucléique cible distincte, dans lequel la région de liaison spécifique à une cible est située à une extrémité 5' de la seconde TSP ;
au moins une région commune de liaison à une amorce ; et
une ou plusieurs copies d'une deuxième région de liaison à une sonde fluorescente (FP),
dans lequel la première partie de la séquence d'acide nucléique cible distincte et la seconde partie de la séquence d'acide nucléique cible distincte sont adjacentes, et
dans lequel un rapport prédéterminé de la ou des copies de la première région de liaison FP à la ou aux copies de la deuxième région de liaison FP indique la séquence d'acide nucléique cible distincte à laquelle la paire est conçue pour se lier.

4. Procédé selon la revendication 1,

(i) dans lequel les FP comprennent des séquences oligonucléotidiques comprenant un ou plusieurs acides nucléiques, des analogues d'acides nucléiques, y compris des acides nucléiques peptidiques (PNA), et des acides nucléiques verrouillés (LNA),
(ii) dans lequel l'essai d'amplification d'acide nucléique est choisi dans la liste constituée d'une amplification en cercle roulant (RCA), d'une amplification en cercle roulant hyper-ramifié (HRCA), et d'une amplification en chaîne par polymérase (PCR), ou
(iii) dans lequel l'échantillon comprend une séquence cible d'acide nucléique non identifiée parmi de multiples candidats.

5. Procédé selon la revendication 1, comprenant en outre (i) l'élimination des TSP qui ne sont pas liées à au moins une partie de la séquence d'acide nucléique après la ligature de l'au moins une des TSP liée à au moins une partie de la séquence d'acide nucléique,

(ii) l'élimination des FP non hybridées aux TSS comprenant l'utilisation d'une colonne de spin de purification, ou
(iii) l'utilisation d'un système de détection de molécule unique (SMD) et la mesure d'un nombre de photons de chaque couleur de fluorescence d'un complexe TSS-FP unique comprenant l'utilisation d'une spectroscopie confocale à illustration cylindrique (CICS).

6. Procédé selon la revendication 5, dans lequel (i) le système SMD comprend une puce microfluidique comprenant :

un matériau silicone perméable aux gaz comprenant du polydiméthylsiloxane (PDMS) ;
une chambre de transport comprenant au moins 2 canaux d'écoulement parallèles, chaque canal d'écoulement ayant une dimension d'environ 5 $\mu$m $\times$ 0,5 $\mu$m (largeur $\times$ hauteur) ; et
un ensemble de filtres à une entrée pour réduire le colmatage de canal d'écoulement,
dans lequel la puce microfluidique est utilisée pour mesurer la fluorescence du complexe TSS-TP, de préférence

dans lequel l'échantillon est conduit à travers la puce microfluidique à l'aide d'une source de pression d'azote, et dans lequel la source de pression d'azote est régulée par une série de régulateurs de gaz de précision, ou

(ii) le nombre de photons de chaque couleur de fluorescence du complexe TSS-FP est mesuré par CICS, et dans lequel la séquence d'acide nucléique est identifiée par un rapport de nombres de photons de fluorescence mesurés.

7. Procédé selon la revendication 1, comprenant en outre :

le chargement d'une pluralité de TSP ligaturées, d'un mélange réactionnel d'amplification d'acide nucléique, et de FP sur un ensemble de réceptacles de réaction distincts, de telle sorte que chaque réceptacle de réaction contient jusqu'à une TSP ligaturée ;
la génération de TSS dans chaque réceptacle de réaction par amplification d'acide nucléique à l'aide de la TSP ligaturée comme matrice ;
la liaison des TSS avec une pluralité de FP marquées différemment dans chaque réceptacle de réaction ;
la mesure d'un rapport de fluorescence dans chacun des réceptacles de réaction ; et
l'identification de la séquence d'acide nucléique sur la base du rapport de fluorescence, de préférence
(i) dans lequel l'échantillon contient une pluralité de séquences d'acides nucléiques ou
(ii) dans lequel l'ensemble de réceptacles de réaction discrets est situé sur une puce microfluidique, ou dans lequel l'ensemble de réceptacles de réaction discrets est constitué d'une pluralité de gouttelettes.

8. Procédé selon la revendication 7, dans lequel (i) la puce comprend en outre :

une chambre d'écoulement microfluidique comprenant un ou plusieurs canaux d'écoulement ; et
une pluralité de picopuits avec des dimensions comprises dans la plage de 100 pL à 10 nL,
dans lequel le ou les canaux d'écoulement sont en contact avec la pluralité de picopuits, de préférence comprenant en outre :

le chargement de la pluralité de TSP ligaturées, du mélange réactionnel d'amplification d'acide nucléique et d'une pluralité de MB marquées différemment dans le ou les canaux d'écoulement de la puce microfluidique, de telle sorte que chacun des picopuits contient jusqu'à une TSP ligaturée ;
l'injection d'un fluide comprenant de l'huile et du polydiméthylsiloxane (PDMS) dans le ou les canaux d'écoulement, mais pas dans la pluralité de picopuits, de telle sorte qu'un puits de réaction numérique est formé ;
la génération de TSS dans chacun de la pluralité de picopuits contenant jusqu'à une TSP ligaturée par amplification d'acide nucléique à l'aide de la TSP ligaturée comme matrice ;
la liaison des TSS avec les MB marquées différemment ;
la mesure d'un rapport de fluorescence ; et
l'identification de la séquence d'acide nucléique sur la base du rapport de fluorescence,

(i) chacune de la pluralité de gouttelettes a un volume compris entre environ 5 pL et 1 nL,
(ii) dans lequel la pluralité de gouttelettes est générée sur une puce microfluidique comprenant :

un module de génération de gouttelettes comprenant des canaux d'écoulement, des microvannes et une jonction de focalisation d'écoulement ; et
un module de mesure de gouttelettes comprenant des canaux d'écoulement, des microvannes et un canal d'étranglement d'écoulement,

(iii) dans lequel un système de microscope confocale personnalisé est utilisé pour mesurer une intensité de fluorescence dans la pluralité de gouttelettes, le microscope confocal personnalisé comprenant :

un laser de 488 nm et un laser de 545 nm ;
une pluralité de miroirs dichroïques pour combiner deux faisceaux laser ;
un objectif de microscope $\times 40$ pour focaliser un faisceau laser et recueillir un signal de fluorescence émis par la pluralité de gouttelettes ;
une pluralité de miroirs dichroïques et de filtres passe-bande pour séparer spectralement un signal d'émission de fluorescence souhaité ; et
au moins 2 photodiodes à avalanche (APD) pour recueillir des données de fluorescence, ou

(iv) comprenant en outre :

le chargement d'un mélange comprenant la pluralité de TSP ligaturées, le mélange réactionnel d'amplification d'acide nucléique, et des FP dans un module de génération de gouttelettes sur la puce ;

le chargement simultané d'un fluide comprenant une huile et un agent tensioactif dans le module de génération de gouttelettes, de telle sorte que le mélange est cisaillé en une pluralité de gouttelettes, et dans lequel chaque gouttelette contient jusqu'à une TSP ligaturée ;

la collecte de la pluralité de gouttelettes ;

l'incubation de la pluralité de gouttelettes collectées sur un thermocycleur et la génération de TSS par amplification d'acide nucléique à l'aide de la TSP ligaturée comme matrice ;

la liaison des TSS avec les FP marquées différemment ;

le chargement de la pluralité de gouttelettes dans un module de mesure de gouttelettes sur la puce ;

la mesure d'un rapport de fluorescence à l'aide d'un système de microscopie confocale personnalisé ; et

l'identification de la séquence d'acide nucléique sur la base du rapport de fluorescence mesuré.

9. Procédé selon la revendication 1, dans lequel chacune des TSP comprend en outre une ou plusieurs copies d'une troisième région de liaison à une sonde fluorescente (FP), dans lequel un rapport prédéterminé de la ou des copies de la première région de liaison FP à la ou aux copies de la deuxième région de liaison FP à la ou aux copies de la troisième région de liaison FP indique la séquence d'acide nucléique cible distincte à laquelle la TSP est conçue pour se lier.

10. Trousse de détection multiplexée d'une séquence d'acide nucléique dans un échantillon comprenant :
une pluralité de sondes oligonucléotidiques spécifiques à une cible (TSP), dans laquelle chacune des TSP est conçue pour se lier à une séquence d'acide nucléique cible distincte, et dans laquelle chacune des TSP comprend :

au moins une région de liaison à une cible conçue pour se lier à au moins une partie de la séquence d'acide nucléique cible distincte ;

au moins une région commune de liaison à une amorce ; et

une ou plusieurs copies d'une première région de liaison à une sonde fluorescente (FP) et une ou plusieurs copies d'une deuxième région de liaison FP, dans laquelle un rapport prédéterminé de la ou des copies de la première région de liaison FP à la ou aux copies de la deuxième région de liaison FP indique la séquence d'acide nucléique cible distincte à laquelle la TSP est conçue pour se lier ; et

des instructions permettant de déterminer l'identité de la séquence d'acide nucléique sur la base d'un rapport de fluorescence mesuré,

dans laquelle les FP comprennent des sondes oligonucléotidiques linéaires marquées avec des fluorophores et des balises moléculaires (MB) qui sont des sondes oligonucléotidiques en forme d'épingle à cheveux marquées avec des fluorophores et des inhibiteurs, et dans laquelle la fluorescence des MB est inhibée à un état natif et rétablie lors de l'hybridation aux TSS.

11. Trousse selon la revendication 10, dans laquelle

(i) chacune des TSP comprend en outre :

une première région de liaison à une cible au niveau d'une première extrémité de la TSP, dans laquelle la première région de liaison à une cible se lie à au moins une partie de la séquence d'acide nucléique cible distincte ; et

une seconde région de liaison à une cible au niveau d'une seconde extrémité de la TSP, dans laquelle la seconde région de liaison à une cible se lie à au moins une partie de la séquence d'acide nucléique cible distincte ou

(ii) la pluralité de sondes oligonucléotidiques spécifiques à une cible (TSP) comprend une pluralité de paires de TSP conçues pour se lier à une séquence d'acide nucléique cible distincte, et dans laquelle chaque paire de la pluralité de paires de TSP comprend :
une première TSP comprenant :

une région de liaison spécifique à une cible conçue pour se lier à au moins une première partie de la séquence d'acide nucléique cible distincte, dans laquelle la première région de liaison spécifique à la cible est située à une extrémité 3' de la première TSP ;
au moins une région commune de liaison à une amorce ; et
une ou plusieurs copies d'une première région de liaison à une sonde fluorescente (FP) ; et
une seconde TSP comprenant :

une région de liaison spécifique à une cible conçue pour se lier à au moins une seconde partie de la séquence d'acide nucléique cible distincte, dans laquelle la région de liaison spécifique à une cible est située à une extrémité 5' de la seconde TSP ;
au moins une région commune de liaison à une amorce ; et
une ou plusieurs copies d'une deuxième région de liaison à une sonde fluorescente (FP),
dans laquelle la première partie de la séquence d'acide nucléique cible distincte et la seconde partie de la séquence d'acide nucléique cible distincte sont adjacentes, et
dans laquelle un rapport prédéterminé de la ou des copies de la première région de liaison FP à la ou aux copies de la deuxième région de liaison FP indique la séquence d'acide nucléique cible distincte à laquelle la paire est conçue pour se lier.

12. Trousse selon la revendication 10, comprenant en outre (i) une amorce qui se lie à la région commune de liaison à une amorce, ou
(ii) des réactifs permettant de réaliser un essai d'amplification d'acide nucléique, de préférence dans laquelle les réactifs permettant de réaliser un essai d'amplification d'acide nucléique sont des réactifs adaptés à la réalisation d'un essai d'amplification en cercle roulant, d'un essai d'amplification en cercle roulant hyper-ramifié, ou d'un essai d'amplification en chaîne par polymérase.

13. Trousse selon la revendication 10, comprenant en outre la première FP et la seconde FP.

14. Trousse selon la revendication 13,
dans laquelle la première FP et la seconde FP comprennent des séquences oligonucléotidiques comprenant un ou plusieurs acides nucléiques, des analogues d'acides nucléiques, y compris des acides nucléiques peptidiques (PNA), et des acides nucléiques verrouillés (LNA).

15. Trousse selon la revendication 10, comprenant en outre une enzyme permettant d'éliminer ou d'inactiver des sondes oligonucléotidiques spécifiques à une cible qui ne se lient pas à l'acide nucléique.

FIG. 1A

FIG. 1A(Continued)

**B**

Target-specific probe (TSP)

| Target binding region | Red FP binding region | Green FP binding region | Common primer-binding region | Red FP binding region | Target binding region |

5'p ═══════════════════════════════════════ 3'

Nucleic acid target

Target-specific hybridization & ligation

Circularized TSP

**C**

| TSPs | Targets | R/G Ratios |
|---|---|---|
| 5'p ══════ 3' | *Proteus mirabilis* (PM) | 0 : 1 |
| 5'p ═══════════ 3' | *Human immunodeficiency virus 1* (HIV-1) | 1 : 2 |
| 5'p ═════════ 3' | *Neisseria gonorrhoeae* (NG) | 1 : 1 |
| 5'p ══════════ 3' | *Chlamydia trachomatis* (CT) | 2 : 1 |
| 5'p ═══════════ 3' | *Treponema pallidum* (TP) | 3 : 1 |
| 5'p ════════ 3' | *Escherichia coli* (EC) | 1 : 0 |

## FIGs 1B and 1C

**FIG. 1D**

EP 3 899 038 B1

FIGs 2A and 2B

**A**

| | HIV-1 | NG | CT | TP |
|---|---|---|---|---|
| Red channel | | | | |
| Green channel | | | | |
| R/G ratio | 0.86 | 1.93 | 3.43 | 5.20 |

**B**

*FIGs 3A and 3B*

**FIGs 4A-4C**

**FIG. 5**

**FIG. 6**

FIG. 7

FIG. 8

**A**

**B**

*FIGs 9A and 9B*

**+TSPs mix**
**+Nucleic acid targets**

Hybridization & Ligation

**+Common primers**
**+Polymerase**

Amplification

**+Fluorescent probes (FPs)**

Fluorescent Probe Hybridization

Two-channel CICS

TSPs mix

Target 1
Target 2

Target 1   Target 2

Common primers

Polymerase

Binding target specific sequences (TSSs) with FPs

Counts

R/G ratios

*FIG. 10*

**FIG. 11**

FIG. 12

**FIG. 13**

**FIG. 14**

FIG. 15

EP 3 899 038 B1

FIGs 16A and 16B

FIG. 17

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62784100

**Non-patent literature cited in the description**

- **YE ZHANG et al.** *Ratiometric Fluorescence Coding for Multiplex Nucleic Acid Chemistry*, 16 October 2018, vol. 90 (8), 12180-12186
- **MORENO, J. G.** ; **CROCE, C. M.** ; **FISCHER, R.** ; **MONNE, M.** ; **VIHKO, P.** ; **MULHOLLAND, S. G.** ; **GOMELLA, L. G.** Detection of Hematogenous Micrometastasis in Patients with Prostate-Cancer. *Cancer Res*, 1992, vol. 52 (21), 6110-6112
- **BERNARD, P. S.** ; **WITTWER, C. T.** Real-time PCR technology for cancer diagnostics. *Clin Chem*, 2002, vol. 48 (8), 1178-1185
- **BOEHM, C. D.** Use of Polymerase Chain-Reaction for Diagnosis of Inherited Disorders. *Clin Chem*, 1989, vol. 35 (9), 1843-1848
- **HE, L. P.** ; **CHINNERY, P. F.** ; **DURHAM, S. E.** ; **BLAKELY, E. L.** ; **WARDELL, T. M.** ; **BORTHWICK, G. M.** ; **TAYLOR, R. W.** ; **TURNBULL, D. M.** Detection and quantification of mitochondrial DNA deletions in individual cells by real-time PCR. *Nucleic Acids Res*, 2002, vol. 30 (14)
- **MONIS, P. T.** ; **GIGLIO, S.** Nucleic acid amplification-based techniques for pathogen detection and identification. *Infect Genet Evol*, 2006, vol. 6 (1), 2-12
- **HIGUCHI, R.** ; **DOLLINGER, G.** ; **WALSH, P. S.** ; **GRIFFITH, R.** Simultaneous Amplification and Detection of Specific DNA-Sequences. *Bio-Technol*, 1992, vol. 10 (4), 413-417
- **WITTWER, C. T.** ; **HERRMANN, M. G.** ; **MOSS, A. A.** ; **RASMUSSEN, R. P.** Continuous fluorescence monitoring of rapid cycle DNA amplification. *Biotechniques*, 1997, vol. 22 (1), 130
- **LANDEGREN, U.** ; **KAISER, R.** ; **SANDERS, J.** ; **HOOD, L.** A Ligase-Mediated Gene Detection Technique. *Science*, 1988, vol. 241 (4869), 1077-1080
- **ABRAVAYA, K.** ; **CARRINO, J. J.** ; **MULDOON, S.** ; **LEE, H. H.** Detection of Point Mutations with a Modified Ligase Chain-Reaction (Gap-Lcr). *Nucleic Acids Res*, 1995, vol. 23 (4), 675-682
- **COMPTON, J.** Nucleic-Acid Sequence-Based Amplification. *Nature*, 1991, vol. 350 (6313), 91-92

- **HEIM, A.** ; **GRUMBACH, I. M.** ; **ZEUKE, S.** ; **TOP, B.** Highly sensitive detection of gene expression of an intronless gene: amplification of mRNA, but not genomic DNA by nucleic acid sequence based amplification (NASBA). *Nucleic Acids Res*, 1998, vol. 26 (9), 2250-2251
- **ERIKSSON, R.** ; **JOBS, M.** ; **EKSTRAND, C.** ; **ULLBERG, M.** ; **HERRMANN, B.** ; **LANDEGREN, U.** ; **NILSSON, M.** ; **BLOMBERG, J.** Multiplex and quantifiable detection of nucleic acid from pathogenic fungi using target-specific probes, generic real time PCR and specific suspension array readout. *J Microbiol Meth*, 2009, vol. 78 (2), 195-202
- **ELENITOBA-JOHNSON, K. S. J.** ; **BOHLING, S. D.** ; **WITTWER, C. T.** ; **KING, T. C.** Multiplex PCR by multicolor fluorimetry and fluorescence melting curve analysis. *Nat Med*, 2001, vol. 7 (2), 249-253
- **LIAO, Y. Q.** ; **WANG, X. B.** ; **SHA, C.** ; **XIA, Z. M.** ; **HUANG, Q. Y.** ; **LI, Q. G.** Combination of fluorescence color and melting temperature as a two-dimensional label for homogeneous multiplex PCR detection. *Nucleic Acids Res*, 2013, vol. 41 (7)
- **SCHOUTEN, J. P.** ; **MCELGUNN, C. J.** ; **WAAIJER, R.** ; **ZWIJNENBURG, D.** ; **DIEPVENS, F.** ; **PALS, G.** Relative quantification of 40 nucleic acid sequences by multiplex ligation-dependent probe amplification. *Nucleic Acids Res*, 2002, vol. 30 (12)
- **CHUNG, B.** ; **SHIN, G. W.** ; **NA, J.** ; **OH, M. H.** ; **JUNG, G. Y.** Multiplex quantitative foodborne pathogen detection using high resolution CE-SSCP coupled stuffer-free multiplex ligation-dependent probe amplification. *Electrophoresis*, 2012, vol. 33 (9-10), 1477-1481
- **HUANG, Q. Y.** ; **ZHENG, L. L.** ; **ZHU, Y. M.** ; **ZHANG, J. F.** ; **WEN, H. X.** ; **HUANG, J. W.** ; **NIU, J. J.** ; **ZHAO, X. L.** ; **LI, Q. G.** Multicolor Combinatorial Probe Coding for Real-Time PCR. *Plos One*, 2011, vol. 6 (1)
- **HUANG, Q. Y.** ; **HU, Q. H.** ; **LI, Q. G.** Identification of 8 foodborne pathogens by multicolor combinational probe coding technology in a single real-time PCR. *Clin Chem*, 2007, vol. 53 (10), 1741-1748

- **RAJAGOPAL, A.** ; **SCHERER, A.** ; **HOMYK, A.** ; **KARTALOV, E.** Supercolor coding methods for large-scale multiplexing of biochemical assays. *Anal Chem*, 2013, vol. 85 (16), 7629-36

- **LIU, D. Y.** ; **DAUBENDIEK, S. L.** ; **ZILLMAN, M. A.** ; **RYAN, K.** ; **KOOL, E. T.** Rolling circle DNA synthesis: Small circular oligonucleotides as efficient templates for DNA polymerases. *J Am Chem Soc*, 1996, vol. 118 (7), 1587-1594

- **LIZARDI, P. M.** ; **HUANG, X. H.** ; **ZHU, Z. R.** ; **BRAY-WARD, P.** ; **THOMAS, D. C.** ; **WARD, D. C.** Mutation detection and single-molecule counting using isothermal rolling-circle amplification. *Nat Genet*, 1998, vol. 19 (3), 225-232

- **SCHWEITZER, B.** ; **WILTSHIRE, S.** ; **LAMBERT, J.** ; **O'MALLEY, S.** ; **KUKANSKIS, K.** ; **ZHU, Z. R.** ; **KINGSMORE, S. F.** ; **LIZARDI, P. M.** ; **WARD, D. C.** Immunoassays with rolling circle DNA amplification: A versatile platform for ultrasensitive antigen detection. *P Natl Acad Sci USA*, 2000, vol. 97 (18), 10113-10119

- **DEAN, F. B.** ; **NELSON, J. R.** ; **GIESLER, T. L.** ; **LASKEN, R. S.** Rapid amplification of plasmid and phage DNA using phi29 DNA polymerase and multiply-primed rolling circle amplification. *Genome Res*, 2001, vol. 11 (6), 1095-1099

- **NIELSEN, P. E.** ; **EGHOLM, M.** An introduction to peptide nucleic acid. *Curr Issues Mol Biol*, 1999, vol. 1 (1-2), 89-104

- **DEMIDOV, V. V.** ; **YAVNILOVICH, M. V.** ; **BELOT-SERKOVSKII, B. P.** ; **FRANKKAMENETSKII, M. D.** ; **NIELSEN, P. E.** Kinetics and Mechanism of Polyamide (Peptide) Nucleic-Acid Binding to Duplex DNA. *P Natl Acad Sci USA*, 1995, vol. 92 (7), 2637-2641

- **ALI, M. M.** ; **LI, F.** ; **ZHANG, Z. Q.** ; **ZHANG, K. X.** ; **KANG, D. K.** ; **ANKRUM, J. A.** ; **LE, X. C.** ; **ZHAO, W. A.** Rolling circle amplification: a versatile tool for chemical biology, materials science and medicine. *Chem Soc Rev*, 2014, vol. 43 (10), 3324-3341

- **DEMIDOV, V. V.** Rolling-circle amplification in DNA diagnostics: the power of simplicity. *Expert Rev Mol Diagn*, 2002, vol. 2 (6), 542-8

- **BERLIER, J. E.** ; **ROTHE, A.** ; **BULLER, G.** ; **BRADFORD, J.** ; **GRAY, D. R.** ; **FILANOSKI, B. J.** ; **TELFORD, W. G.** ; **YUE, S.** ; **LIU, J.** ; **CHEUNG, C. Y.** Quantitative comparison of long-wavelength Alexa Fluor dyes to Cy dyes: fluorescence of the dyes and their bioconjugates. *J Histochem Cytochem*, 2003, vol. 51 (12), 1699-712

- **ANDERSON, G. P.** ; **NERURKAR, N. L.** Improved fluoroimmunoassays using the dye Alexa Fluor 647 with the RAPTOR, a fiber optic biosensor. *J Immunol Methods*, 2002, vol. 271 (1-2), 17-24

- **HSIEH, C. C.** ; **DOYLE, P. S.** Studying confined polymers using single-molecule DNA experiments. *Korea-Aust Rheol J*, 2008, vol. 20 (3), 127-142

- **ARAKI, S.** ; **NAKAI, T.** ; **HIZUME, K.** ; **TAKEYASU, K.** ; **YOSHIKAWA, K.** Hydrodynamic radius of circular DNA is larger than that of linear DNA. *Chem Phys Lett*, 2006, vol. 418 (1-3), 255-259

- **LI, H. T.** ; **YING, L. M.** ; **GREEN, J. J.** ; **BALASU-BRAMANIAN, S.** ; **KLENERMAN, D.** Ultrasensitive coincidence fluorescence detection of single DNA molecules. *Anal Chem*, 2003, vol. 75 (7), 1664-1670

- **LIU, K. J.** ; **WANG, T. H.** Cylindrical illumination confocal spectroscopy: Rectifying the limitations of confocal single molecule spectroscopy through one-dimensional beam shaping. *Biophys J*, 2008, vol. 95 (6), 2964-2975

- **LARSSON, C.** ; **KOCH, J.** ; **NYGREN, A.** ; **JANS-SEN, G.** ; **RAAP, A. K.** ; **LANDEGREN, U.** ; **NILSSON, M.** *Nat Methods*, 2004, vol. 1, 227-232

- **SHAPOSHNIKOV, S.** ; **LARSSON, C.** ; **HENRIKS-SON, S.** ; **COLLINS, A.** ; **NILSSON, M.** *Mutagenesis*, 2006, vol. 21, 243-247

- **LIZARDI, P. M.** ; **HUANG, X. H.** ; **ZHU, Z. R.** ; **BRAY-WARD, P.** ; **THOMAS, D. C.** ; **WARD, D. C.** *Nat Genet*, 1998, vol. 19, 225-232

- **MIGNARDI, M.** ; **MEZGER, A.** ; **QIAN, X. Y.** ; **LA FLEUR, L.** ; **BOTLING, J.** ; **LARSSON, C.** ; **NILSSON, M.** *Nucleic Acids Res*, 2015, vol. 43

- **HARDENBOL, P.** ; **BANER, J.** ; **JAIN, M.** ; **NILSSON, M.** ; **NAMSARAEV, E. A.** ; **NEUMANN, G. A.** ; **FAKHRAI-RAD, H.** ; **RONAGHI, M.** ; **WILLIS, T. D.** ; **LANDEGREN, U.** *Nat Biotechnol*, 2003, vol. 21, 673-678